# EUROPEAN PATENT APPLICATION

(11) **EP 1 972 687 A1**
(43) Date of publication of application: **24.09.2008**
(21) Application number: 07290357.8
(22) Date of filing: 23.03.2007
(51) Int. Cl.: C12N 9/64, A61P 7/02, C12Q 1/68, A61K 38/48

(54) **Polynucleotides and polypeptides of human factor VII gene, SNPs**

(71) Applicant: GenOdyssee, 91058 Evry Cedex (FR)
(72) Inventor: Escary, Jean-Louis, 78150 Le Chesnay (FR)
(74) Representative: Caen, Thierry Alain

(57) **Abstract**

The present invention relates to new polynucleotides deriving from the nucleotide sequence of Factor VII (FVII) gene comprising new natural SNPs, new polypeptides encoded by said polynucleotides as well as their therapeutic uses.

SEQUENCE LISTING

<110>  GENODYSSEE

<120>  NEW POLYNUCLEOTIDES AND POLYPEPTIDES OF THE FACTOR VII GENE

<130>  BIF 117157

<160>  10

<170>  PatentIn version 3.1

<210>  1

<211>  14241

<212>  DNA

<213>  Homo sapiens

<220>

<221>  Factor VII gene sequence

<222>  (1)..(13218)

<223>

<400>  1
agtcccatgg ggaatgtcaa caggcagggg cagcactgca gagatttcat catggtctcc      60

caggccctca ggctcctctg ccttctgctt gggcttcagg gctgcctggc tgcaggtgcg     120

tccggggagg ttttctccat aaacttggtg gaagggcagt gggcaaatcc aggagccagc     180

ccgggcttcc caaaccccgc ccttgctccg gacaccccca tccaccagga gggttttctg     240

gcggctcctg ttcaatttct ttccttctag aaaccagcat ccaggcacag gaggggaggc     300

ccttcttggt ggcccaggct ttggcgggat tatttttcaa agaactttag gagtgggtgg     360

tgctttcctg gcccccatgg gcccctgcct gtgaggtcgg acaagcgcag ggagtctggg     420

gcctctcaga gtgcaggaag tgcgcacagg gtgctcccag gctggggagc acaggtaggg     480

gacggtgcgt gggggatggc gcctggggca tgggggatgg ggtgtgggaa acggcatgtg     540

gggcgtaggg gatggggtgt ggaggatcgg gggtggggat ggcgtgtggg gtgtggggga     600

tgggccgtgg gggggtgggg cctgggaaac agcatgtggg gcatggggtg tgggggtgag     660

gtgtgggaaa gtgtgtgggg tgtgggggat ggggcatgga aagggcgtgt ggggtgcagg 720
ggatggggca tggaggtgtg ggggatgggg tgtgtggggt gtcggggatg gggcatgtgg 780
ggtgtggggg atggggcatg gaaagggcgt gtggggtgca gaggatgggg catggggggg 840
tggggatggc gagtggggct ggggcctggg aatggtgagt ggggcatggg gatggcgagt 900
agggggtgtg gcgtgaggat ggctagtggg gcgtggggat ggcgtgtggg gatggcgagt 960
ggggggtggg ctgtgaggga cagtgcctgg gatgtggggc tgcagcccta gctcacagca 1020
tggccttatg accccggcca ccttcctgcc ccaggcgggg tcgctaaggc ctcaggagga 1080
gaaacacggg acatgccgtg gaagccgggg cctcacagag gtgagcaggg actgccactg 1140
gttttgtcct ggggcccagt gggggccaac atcacctcct tcccctccca tggcaaagag 1200
ccagcccgcg gggtggctac tgcagtgccc cccaaggagg gtgttccctg ctcgagagga 1260
agtgaccgct ccagcttggc cttccctggg actggggtgc aggcgatttt atcttctttg 1320
ctccattctg ttccttccag ataatcgtgt gttcttcatc aggttttcct cagttcttga 1380
gagcttttct gatgcaaatc tgctttcacc ccagggcggt caccggctct gctcacacca 1440
gcctccaagg gtgtgggtgt cccgggagtg tgggtgtccc gggggcgtgg gtgtcccagg 1500
agtgtgggtg tcccgggggc gtgggtgtcc cgggagtgtg ggtgtcccgg gggcgtgggt 1560
gtcccgggag tgtgggtgtc ccggaggcga gggtgtcccg ggagtgtggg tgtcccgggg 1620
gagtgggtgt cccgggagtg tgggtgtccc ggaggcgagg gtgtcccggg agtgtgggtg 1680
tcccgggggc gtgggtgtcc cgggagtgtg ggtgtcccgg gggagtgggt gtcccgggag 1740
tgtgggtgtc ccggaggcga gggtgtcccg ggagtgtggg tgtcccgggg gagtgggtgt 1800
cccgggagtg tgggtgtccc ggaggcgagg gtgtcccggg agtgtgggtg tcccgggggc 1860
gtgggtgtcc cgggagcgtg ggtgtcccgg gggcgtgggt gtcccgggag tgtgggtgtc 1920
ccgggggcgt gggtgtcccg ggagtgtggg tgtcccgggg gcgtgggtgt cccgggagtg 1980
tgggtgtccc gggagtgtgg gtgttccgga ggcgagggtg tcccgggagt gtgcgtgtcc 2040
cgggggcgtg ggtgtcccgg gggcgtgggt gtcccggggg cgtgggtgtt ccggaggcga 2100
gggtatccca gaagtgtgag tgtcccaggg gcgtgggtgt cccgggggtg tgggtgtccc 2160
gggggcgtgg gtgtcccggg agtgtgggtg ttccggaggt gagggtgtcc cgggagtgtg 2220
ggtgttccgg aggcgagggt gtcccgggag tgtgggtgtc ccgggggcgt gggtgtcccg 2280
ggagtgtggg tgttccggag gtgagggtgt cccgggagtg tgggtgttcc ggaggcgagg 2340
gtgtcccggg agtgtgggtg tcccaggggc gtgggtgtcc cgggagtgtg ggtgttccgg 2400

aggcgagggt gtcccgggag tgtgggtgtt ccggaggcga gggtgtcccg ggagtgtggg 2460
tgtcccgggg gcgtgggtgt cccgggggtt gtgggtgtcc cgggagtgtg ggtgttccgg 2520
aggcgagggt gtcccgggag tgtgggtgtt ccggaggcga gggtgtcccg ggagtgtggg 2580
tgtcccgggg gtgtgggtgt cccgggggtg tgggtgtccc gggagtgtgg gtgtcccggg 2640
ggagtgggtg tcccgggagt gtgggtgttc cggaggcgag ggtgtcccag gagcgtgggt 2700
gtcccggagg cgagggtgtc ccgggagcgt gggtgtcccg ggggcgtggg tgtcccggga 2760
gtgtgggtgt cccgggggag tgggtgtccc gggagtgtgg gtgtcccgga ggcgagggtg 2820
tcccaggagt gtgggtgtcc cgggggcgtg ggtgtcccgg gagtgtgggt gttccagagg 2880
cgagggtatc ccagaagtgt gagtgtcccg ggggtgtggg tgtcccgggg gtcgtgggtg 2940
tcccgggagt gtgggtgttc cagaggcgag ggtgtcccgg gagtgtgggt gtcccagggg 3000
tgtgggtgtc ccgggggcgt gggtgtcccg ggagtgtggg tgtcccgggg gagtgggtgt 3060
cccgggagtg tgggtgttcc ggaggcgagg gtgtcccggg agtgtgggtg ttccggaggc 3120
gagggtgtcc cgggagcgtg ggtgtcccgg gggcgtgggt gtcccgggag cgtgggtgtc 3180
ccaggggtgt gggtgtcccg ggggcgtggg tgtcccggga gtgtgggtgt cccgggggag 3240
tggatgtccc gggagtgtgg gtgttccgga ggcgagggtg tcccgggagt gtgggtgttc 3300
cggaggcgag ggtgtcccgg gagtgtgggt gtcccggggg cgtgggtgtc ccgggagtgt 3360
gggtgtcccg ggggcgtggg tatcccagaa gtgtgagtgt cccaggggcg tgggtgtccg 3420
gggggcgtgg gtgtcccggg ggtgtgggtg tcccgggggt cgtgggtgtc ccgggagcgt 3480
gggtgtcggg gactgcaggg acatgggcct cccctcccac tcctgccgcc cagggcacct 3540
cctgtgagga ctcggagtcc gtgagttccc acctccttga gcccgattct ttggtgtccc 3600
cgcctgcatc ctcagcctcc ttccaaacca gaccagttct ctaggggcgt cgacgtgtga 3660
aactgatttt aaagaaaaca ggcagtggcc tttctctcgg ccccacgtgg cccagtagcg 3720
ctcaccttcc gtcccttctt ccgcgctcag taaccaattt aggccgctcc tgcagaactc 3780
gggctcctgc ccaccggccc acagcgtcca cctgaggcct cgtcctccca gcaaaggtcg 3840
tccctccgga acgcgcctcc tgcggcctct ccagagcccc tcccgcgcgt cctctcagcc 3900
ccgctcgcct cctcccgggg cctccctctc ccgcctgccc ccaggcccgt ctcccctcgc 3960
gggctgaggc aggttcgggc agcacggccg ccccggggcg ggggtcactc tccaccaccg 4020
cgtggtgccc acagctcacg gcgctcccgg gtgacggtcc cctcggctgt agggcgtcct 4080
gaagagcggc ctgctcggag ctgagcgcac ggggttgcct cgccctgggc gtctctggcc 4140
ctcaccagcc ccgtcttccc atgggcaaaa cggcggtcct gtttgtccac aagtaaccgt 4200

cggggttacg gaggggccag gagctgcggc ggggggctgt gctctcagga ccggccccag 4260
gaggatccgc gcgaggtctg gagctctcag gggtcgcggg ggacagaggg gccccaagcg 4320
gaggcgggga aggcggcaga agcccaggac cgccaagagc tggcgaggaa gcccggggct 4380
cgctgtcggg ggagccgggc aggggccgcg cctcggacca ggacggaggc ctggggaagg 4440
cggatctggc cgccggagac gcggtgcggg tggagacgag ggatttggat ttccgcgggc 4500
ggctgtacgg atttccacgc gcggttcacg tgggccccag ggggttgccc ggcacccggg 4560
gccgcgccgc cttctcctcg ccggcatcga cccgcagcct cacgtttacg cggcggcgcc 4620
cgcagccccc ttcggcccgg cttccgcgcg tgcccccgag cgcgccctcg ggatcagccc 4680
ccggaagcag agaggccagg ccgggaagga tgggcgacgg gggtggctga cccgggagca 4740
cggcagggag gacacccagc caggcccgcg agcagcgccg ctcccctcct ccaggacggg 4800
cgggaacctg cgatgccccc gccgcgtggg ccgtggggcg gtctccgagg cactgggcgg 4860
ggcacgcggt gggcgcttca cggaactcgc atttcccagt cttcgtaacc caggaggaag 4920
cccacggcgt cctgcaccgg cgccggcgcg ccaacgcgtt cctggaggag ctgcggccgg 4980
gctccctgga gagggagtgc aaggaggagc agtgctcctt cgaggaggcc cgggagatct 5040
tcaaggacgc ggagaggacg gtgagcccag cctcggggcg ccccgcgccg cggacactgc 5100
aggcggcggt gaaccaggcc gcgtggggcc gcctgcgtct ctttggctgc ggctgtgggc 5160
ggcgaacacg cagcggcgcc cgcgcggcgc tttctgcggg ggtcgctttc cgcccggggt 5220
gactccgctt tcctgggcga tgccccccac ccccaggcac gcgctctccc cgtgcggccg 5280
caccgcgcat gccggttttc acatcagaaa atacgatttg caaagcacac ttagggtgtc 5340
ccccttaact tcccaaggga gtccccccag tccccgaagg gtccagggca gcctgcgcat 5400
cgcagacgcg cgcggctcgc agaagggacg tggtgagaag ctggcccaca gcatgccacc 5460
agcggcacct cctcagggca cgtgtcgggg agaaacaaca cttagggacc tgggactttc 5520
tccagctcac gctcacgggt cacctcacac tccaagatca cctcaaagag gacacctcac 5580
acagggcaca cttcacactc acaggtcacc tcacactcac aggacacctc acactcacag 5640
ggcacacttc acactcacgg gtcacctcac actccaagat cacctcaaag aggacacctc 5700
acacagggca cacttcacac tcacaggtca cctcacactc acaggacacc tcacactcac 5760
agggcacact tcacactcac gggtcacctc acactccaag atcacctcaa agaggacacc 5820
tcacacaggg cacacttcac actcacgggt cacctcacac tcacaggaca cctcacacaa 5880
gacacctcac acggggcaca cttcacactc acaggtcacc tcacacccac aggacacctc 5940

acacagggca cacttcacac tcacgggtca cctcacactc acaggacacc tcacacaaga 6000
cacctcacac ggggcacact tcacactcac aggtcacctc acacccacag gacacctcac 6060
acagggcaca cttcacactc acgggtcacc tcacactcac aggacacctc acactcaggg 6120
cgcacttcac actcacgggt cacctcacac ccacaggaca cctcacagag gtcacctcac 6180
acaggacacc tcacactcag ggtgcacttc aaacccacag gtcatttcac ctcacactca 6240
caggacacct cacacaagat accacacggg gcacacttca cactcacagg tcacctcaca 6300
ctcacaggac acctcacaga ggtcacctca cacggggcac acttcacact cacaggtcac 6360
ctcacaccca caggacacct cacagaggtc acctcacacc cacaggacac ctcacacagg 6420
acacctcaca gaggtcacct cacacccaca ggacacctca cactcatagg tcacctcagt 6480
cttacaggac aactcacact cacaggtcac cttactctca caggacacct cacactcaca 6540
ggtcacctta ctctcacagg acacctcact ctcacaggac acctcacaca gggcacactt 6600
cactcccaca ggtcaccata cctcacacag atcacctcat actcacagat cacttcattc 6660
tcacaggata cctcacactc agggcacact tcacactcac aggtcacacc tcacacagat 6720
catctcattc tcacaggaca cctccctctc acaggtcacc tcacactcac aggacacctc 6780
acagaggtca cctcacaccc acaggacacc tcacagaggt cacctcacac ggggcacact 6840
tcacactcag gtcacctcac acccacagga cacctcacag aggtcacctc acacccacag 6900
gacaactcac agaggtcacc tcacacagga cacctcacaa aggtcacctc acacccacag 6960
gacacctcac actcataggt cacctcagtc ttacaggaca actcacactc acaggtcacc 7020
ttactctcac aggacacctc acactcacag gtcaccttac tctcacagga cacctcacac 7080
agggcacact tcactcccac aggtcaccat acctcacaca gatcacctca tactcacaga 7140
tcacttcatt ctcacaggat acctcacact cagggcacac ttcacactca caggtcacac 7200
ctcacacaga tcatctcatt ctcacaggac acctccctct cacaggtcac cttacactca 7260
tctcacactc acaggtcgcc acacctcaca ctcacaggat gcctcacact cacagaacca 7320
catctcatat gcacaagaca cctcacactc aggacacctc atgctcaaag aagcctcaca 7380
ctcacaggag gtccagctgt ctgaggcaaa ggctaacatg accctttcca gacaaattga 7440
ggatggtcat gcctagcatt tttatacacc tagttttgaa agcatttctc atctgttgta 7500
ttctcacagc accccgtgag tttaagttca ggtggccaac agtttcttca gcaatcactt 7560
ttttctgtgg agtgcttttg ctgtttgtgg aatattttgc atctgctact gcaccctctc 7620
cccgtatgtg tggccaccct gtcagaggtg gagctgtggc tcagagcctg tgtacctcgt 7680
cccaggtcca cagctcagcg acagaagagt cagggttgaa cctcgggtgt tctgacttgg 7740

gagcaggaaa tgtgtggtca cccatagttc cagatgtcct ggggaggggc caagattaga 7800
agaaacctac ctcagctcca gaggaaagtc tggcttcctg agcccacccc gccagaccca 7860
ggtccaagtc ccccaacccc agttcatggt gtgtccagtg cttaccgttg ggtgctctgg 7920
tgaaggtgca tctcacgagg cttgctctct tgttccttca gaagctgttc tggatttctt 7980
acagtggtga gtggatgatc accaccagtc ctgcctgcaa cccttctcag cttactgaca 8040
ccagcccact ccacagatgg ggaccagtgt gcctcaagtc catgccagaa tgggggctcc 8100
tgcaaggacc agctccagtc ctatatctgc ttctgcctcc ctgccttcga gggccggaac 8160
tgtgagacgc gtaaggcccc actttgggtc ccatatttgc agagggccct ggggagctgg 8220
tggaggtggc ctggccaacc gggctgcagg gtgcacaacc tggtggggtg tgtaggccgg 8280
gcattcaggg ctcagcccca gttggaaatt ggtctaggtg accttgaaat cccttccagt 8340
ctgaggtctt tgacagggac ccaaggttct gattatcaga ctcagtggcc ccttgggctc 8400
ccggccctgg gcaattctca gccctcgaga tggcccagct gagagtccct gtgtccctgt 8460
cccacttcca catcccacca cgcaggaccg cttggtaaac ttccccttct ctactttcca 8520
ttacaaaggt ttgaggggtt tgtttttttt ttaaccatct gaatattaaa ttatcacaaa 8580
gtttgaggcc cccaacctcc cttgggttca gtaattcact agaaggactc atagaatcca 8640
ctgaagtgga tacactcaca ggtaccgttt attacagcaa aggatgcagg cttaagtctg 8700
cagagggacc aggcacaagc ttccccttgt cctctccctg tggggtcatg tggacagtcc 8760
ttaattctcc cagaatgacg tgtgacgaga cgtgggaagt actgccaact tgggaagctc 8820
tacgagcccc ggtgtccaga ggttttatca gggctcaatc acatagaccc agctgaccac 8880
ccgcatggct gacctcagtc tcagcccctc cagaggctac gccgatagtg cggcccaagg 8940
ccccaccata catcacattg tcagctagac catccagcat ggctcaaggc ccaggtaaac 9000
accaacattc cctcaggcaa gaccttccaa gggcttagcg gtcatttccc aggagccaag 9060
gcaaaggcta ccctttctct ggcacagcag ttcatccttg accacccaag accacattct 9120
tacactgaat gagctctcct gtgcagcagc cattttcttc tctaagcaga agagagccca 9180
gcaagctgga ggaggctgaa gagagaggct tcctgctggt catctgggtc cagaatgcct 9240
ggagatctct gctcagccct ggtgcccagc agccctggtg tgcatcctgc agggcaggcc 9300
ttcccgccgg agtcctggac ttgctcaggg ccactcccct tgcccatgtc aaccaaagtc 9360
aggctgccgg ttctgcttct tctgtctgag cccatgacca gtgctgggac taactgtccc 9420
caggcgggct cacggtggta cgaggccagc ttggagaact gtctcagctc tctggtcctc 9480

tcgtcagttg ggtctctgat tggaaagtcc cttggacact tttaccatcc ccattggact 9540
ttcactttcc cccaggctcc catcagctgc tcggaagagt ggtcaccctg gaggccactg 9600
cccaccagcc aggcaccccc caaatgcaac cgcagccagc actgccagcc actggcaagg 9660
ctgttcagac atgtggctcc tctgatccac gccttgtcct ttggatcagt ccacggagca 9720
ggtggtgcca agctcaggct ctgtcaccca cagctcagtg ccaccttcca ggcagaacac 9780
cactgctgac ccagggcatg gccaccccgg gggctggctc tcgctgaccc ccagaagccc 9840
ctctcagggt gtccccttcc tgtccccaga caaggatgac cagctgatct gtgtgaacga 9900
gaacggcggc tgtgagcagt actgcagtga ccacacgggc accaagcgct cctgtcggtg 9960
ccacgagggg tactctctgc tggcagacgg ggtgtcctgc acacccacag gtgaccaggc 10020
ttcatgtccc agtcccagat gacaccagtc cctgtcccac tacggattat cttactggac 10080
aaaagacggg tgggagtggc ttcacatcta ctgagcacta actatgcact gaccaattgt 10140
gaggtgggat ctgggcacca agggtggcac aggccagagc gacagtgact aggatgggca 10200
ccctgggggc aatccctgaa tggcctcagg ccccctgcca attctaggca gaccagggga 10260
gccaagcaag gcactatctc acgtccaact cccactcgca ggacctccgc cagggttcat 10320
gaatctactt cggcacagcc aatgtctgta ctgactgctg cccactctgc attccaaaac 10380
tcgtaaaggc tcctgggaaa atgggatgtt tctccaaacc agcctggaac gaatgggctg 10440
cacttccaaa agcagggaca ccccacaccc actgtgtctc aaagaggcgg acgtgcccac 10500
cctggccaca cagcctggga ctcagcctgc cacctcctcg ggcttccttt ctggcccaag 10560
accttgattg aagcagatca aaactaagca tgggatcaaa acaacacagt ttgattcatc 10620
tttaggtaga atttcattca ccttctacta aagtcaaaca acacatcttc tccctgaaaa 10680
gtgagcagag ggcggtttta agacgtaagc cctctgtttc ctccaaaacc agccctgacc 10740
attgtctcct cagccagcca cttcttcaag ggcctctcat ggccgggccc caccagtcag 10800
gcccagccga ggccctgcct tccaccaccc ctgggccctg ggagctcctg ctcctggggg 10860
cctcccatag cctcggcctc aaggcctctc agaggatggg tgtttctgaa tctttcctag 10920
tggcacgttc atccctcaca aatctctgca tctttctgac ttttgtttta cacagttgaa 10980
tatccatgtg gaaaaatacc tattctagaa aaaagaaatg ccagcaaacc ccaaggccga 11040
attgtggggg gcaaggtgtg ccccaaaggg gagtgtccat ggcaggtaag gcttcccctg 11100
gcttcaggat tccaagccct gagggtcttg aagccttttg aatgtgaaca acagctctgg 11160
aagggaaaat gggcaggtca gccccaagcc caccaggctc caagtcagca cacctagcac 11220
ctccagctcg cggcaccccc atgcttttag tggggcaagg aaggagaaaa gaaaacgaca 11280

ctcactgagg gtctaccctg tgcagagaac cctgcgagat gccccatccg agttgtcacg 11340
tcgtcctcac ggttactctt tgaggtggga tctttgcctg atctttgcaa aatcaggagc 11400
attggatcaa agctatgtga agatcctgtg aggtgaacag tgaaatctca cagcgacatt 11460
tgtattcttg ggccgtgccc aagagcacgt ctcggctaga gaggggcaca gcctcccaga 11520
gccaggtctg agcagctttg cctgggaggg atctgcaaag accccaggat ttcagaaaga 11580
aattgtgcaa tgccagaggt tccttggcat gcccgggagg gcgagtcatc agagaaacaa 11640
tgacagcaat gtgacttcca cacctcctgt ccccccgccc aggtcctgtt gttggtgaat 11700
ggagctcagt tgtgtggggg gaccctgatc aacaccatct gggtggtctc cgcggcccac 11760
tgtttcgaca aaatcaagaa ctggaggaac ctgatcgcgg tgctgggtgg gtaccactct 11820
cccctgtccg accgcggtgc tgggtgggtg ccactcttcc ctgtccgacc gcggtgctgg 11880
gtgggtgcca ctctcccctg tccgaccgcg gtgctgggtg ggtgccactc tcccctgtcc 11940
gaccgcggtg ctgggtgggt gccactctcc gctgtccgac cgcggtgctg ggtgggtacc 12000
actctcccct gtctgaccgc agctctcaag tgtctcaggg gctgtggctc tgggcttcgt 12060
gctgtcactt ccacagacag acagacatcc ccaaaagggg agcaaccatg ctgggcacga 12120
ctgctgtggc caccgtgctc tcagccactt tcccatgccc aaataaaacg ataaaagact 12180
gggggcttct gcccatcctg cctcacttga ccaagagccc agaagaggat gcgacaccca 12240
gggcctcatg ggaccaccgg ctggcagggg ttctgctcac tgggtttatg ggtgagacga 12300
gcactcccag gagggccact gggccgggaa gaactgtgga gaatcggggc acgccctgtc 12360
ctcccagctg ccagggcaca gcatcccttc cccacctcaa cacccagacc ccagattcac 12420
cccagttcac ttgtccccac acgagccaca ggctgccacc tggggcaggc tggccccacc 12480
ttggggttag atgcaggtcc ccttgcccca gaaggagact gcagcccctg cagacctaga 12540
aatggccaca gcccatcccc atgcaccagg gggtgaggtg gcaggtggtg gaaagggcct 12600
gaggggggct tcttccttcc aggcgagcac gacctcagcg agcacgacgg ggatgagcag 12660
agccggcggg tggcgcaggt catcatcccc agcacgtacg tcccgggcac caccaaccac 12720
gacatcgcgc tgctccgcct gcaccagccc gtggtcctca ctgaccatgt ggtgcccctc 12780
tgcctgcccg aacggacgtt ctctgagagg acgctggcct tcgtgcgctt ctcattggtc 12840
agcggctggg gccagctgct ggaccgtggc gccacggccc tggagctcat ggtcctcaac 12900
gtgccccggc tgatgaccca ggactgcctg cagcagtcac ggaaggtggg agactcccca 12960
aatatcacgg agtacatgtt ctgtgccggc tactcggatg gcagcaagga ctcctgcaag 13020

ggggacagtg gaggcccaca tgccacccac taccggggca cgtggtacct gacgggcatc 13080

gtcagctggg gccagggctg cgcaaccgtg ggccactttg gggtgtacac cagggtctcc 13140

cagtacatcg agtggctgca aaagctcatg cgctcagagc cacgcccagg agtcctcctg 13200

cgagccccat ttccctagcc cagcagccct ggcctgtgga gagaaagcca aggctgcgtc 13260

gaactgtcct ggcaccaaat cccatatatt cttctgcagt taatggggta gaggagggca 13320

tgggagggag ggagaggtgg ggagggagac agagacagaa acagagagag acagagacag 13380

agagagactg agggagagac tctgaggaca tggagagaga ctcaaagaga ctccaagatt 13440

caaagagact aatagagaca cagagatgga atagaaaaga tgagaggcag aggcagacag 13500

gcgctggaca gaggggcagg ggagtgccaa ggttgtcctg gaggcagaca gcccagctga 13560

gcctccttac ctcccttcag ccaagcccac ctgcacgtga tctgctggcc tcaggctgct 13620

gctctgcctt cattgctgga gacagtagag gcatgaacac acatggatgc acacacacac 13680

acgccaatgc acacacacag agatatgcac acacacggat gcacacacag atggtcacac 13740

agagatacgc aaacacaccg atgcacacgc acatagagat atgcacacac agatgcacac 13800

acagatatac acatggatgc acgcacatgc caatgcacgc acacatcagt gcacacggat 13860

gcacagagat atgcacacac cgatgtgcgc acacacagat atgcacacac atggatgagc 13920

acacacacac caatgcgcac acacaccgat gtacacacac agatgcacac acagatgcac 13980

acacaccgat gctgactcca tgtgtgctgt cctctgaagg cggttgttta gctctcactt 14040

ttctggttct tatccattat catcttcact tcagacaatt cagaagcatc accatgcatg 14100

gtggcgaatg cccccaaact ctcccccaaa tgtatttctc ccttcgctgg gtgccgggct 14160

gcacagacta ttccccacct gcttcccagc ttcacaataa acggctgcgt ctcctccgca 14220

cacctgtggt gcctgccacc c 14241

<210> 2

<211> 1401

<212> DNA

<213> Homo sapiens

<220>

<221> Factor VII gene coding sequence

<222> (1)..(1401)

<223>

<400> 2
atggtctccc aggccctcag gctcctctgc cttctgcttg ggcttcaggg ctgcctggct 60

gcaggcgggg tcgctaaggc ctcaggagga gaaacacggg acatgccgtg gaagccgggg 120

cctcacagag tcttcgtaac ccaggaggaa gcccacggcg tcctgcaccg gcgccggcgc 180

gccaacgcgt tcctggagga gctgcggccg ggctccctgg agagggagtg caaggaggag 240

cagtgctcct tcgaggaggc ccgggagatc ttcaaggacg cggagaggac gaagctgttc 300

tggatttctt acagtgatgg ggaccagtgt gcctcaagtc catgccagaa tgggggctcc 360

tgcaaggacc agctccagtc ctatatctgc ttctgcctcc ctgccttcga gggccggaac 420

tgtgagacgc acaaggatga ccagctgatc tgtgtgaacg agaacggcgg ctgtgagcag 480

tactgcagtg accacacggg caccaagcgc tcctgtcggt gccacgaggg gtactctctg 540

ctggcagacg gggtgtcctg cacacccaca gttgaatatc catgtggaaa aatacctatt 600

ctagaaaaaa gaaatgccag caaaccccaa ggccgaattg tggggggcaa ggtgtgcccc 660

aaaggggagt gtccatggca ggtcctgttg ttggtgaatg gagctcagtt gtgtgggggg 720

accctgatca acaccatctg ggtggtctcc gcggcccact gtttcgacaa aatcaagaac 780

tggaggaacc tgatcgcggt gctgggcgag cacgacctca gcgagcacga cggggatgag 840

cagagccggc gggtggcgca ggtcatcatc cccagcacgt acgtcccggg caccaccaac 900

cacgacatcg cgctgctccg cctgcaccag cccgtggtcc tcactgacca tgtggtgccc 960

ctctgcctgc ccgaacggac gttctctgag aggacgctgg ccttcgtgcg cttctcattg 1020

gtcagcggct ggggccagct gctggaccgt ggcgccacgg ccctggagct catggtcctc 1080

aacgtgcccc ggctgatgac ccaggactgc ctgcagcagt cacggaaggt gggagactcc 1140

ccaaatatca cggagtacat gttctgtgcc ggctactcgg atggcagcaa ggactcctgc 1200

aagggggaca gtggaggccc acatgccacc cactaccggg gcacgtggta cctgacgggc 1260

atcgtcagct ggggccaggg ctgcgcaacc gtgggccact ttggggtgta caccagggtc 1320

tcccagtaca tcgagtggct gcaaaagctc atgcgctcag agccacgccc aggagtcctc 1380

ctgcgagccc catttcccta g 1401

<210> 3

<211> 466

<212> PRT

<213>  Homo sapiens

<220>

<221>  Immature Factor VII protein

<222>  (1)..(466)

<223>

<400>  3

<210> 4

<211> 406

<212> PRT

<213> Homo sapiens

<220>

<221> Mature Factor VII protein

<222> (1)..(406)

<223>

<400> 4

<210>  5
<211>  406

<212>  PRT

<213>  Homo sapiens


<220>

<221>  Mature Factor VII protein including S111F
 SNP
<222>  (1)..(406)

<223>


<400>  5


Ala Asn Ala Phe Leu Glu Glu Leu Arg Pro Gly Ser Leu Glu Arg Glu
1               5                   10                  15


Cys Lys Glu Glu Gln Cys Ser Phe Glu Glu Ala Arg Glu Ile Phe Lys
            20                  25                  30


Asp Ala Glu Arg Thr Lys Leu Phe Trp Ile Ser Tyr Ser Asp Gly Asp
        35                  40                  45


Gln Cys Ala Ser Ser Pro Cys Gln Asn Gly Gly Ser Cys Lys Asp Gln
    50                  55                  60


Leu Gln Ser Tyr Ile Cys Phe Cys Leu Pro Ala Phe Glu Gly Arg Asn
65                  70                  75                  80

Cys Glu Thr His Lys Asp Asp Gln Leu Ile Cys Val Asn Glu Asn Gly
                85                  90                  95

Gly Cys Glu Gln Tyr Cys Ser Asp His Thr Gly Thr Lys Arg Phe Cys
            100                 105                 110

Arg Cys His Glu Gly Tyr Ser Leu Leu Ala Asp Gly Val Ser Cys Thr
        115                 120                 125

Pro Thr Val Glu Tyr Pro Cys Gly Lys Ile Pro Ile Leu Glu Lys Arg
    130                 135                 140

Asn Ala Ser Lys Pro Gln Gly Arg Ile Val Gly Gly Lys Val Cys Pro
145                 150                 155                 160

Lys Gly Glu Cys Pro Trp Gln Val Leu Leu Leu Val Asn Gly Ala Gln
                165                 170                 175

Leu Cys Gly Gly Thr Leu Ile Asn Thr Ile Trp Val Val Ser Ala Ala
            180                 185                 190

His Cys Phe Asp Lys Ile Lys Asn Trp Arg Asn Leu Ile Ala Val Leu
        195                 200                 205

Gly Glu His Asp Leu Ser Glu His Asp Gly Asp Glu Gln Ser Arg Arg
    210                 215                 220

Val Ala Gln Val Ile Ile Pro Ser Thr Tyr Val Pro Gly Thr Thr Asn
225                 230                 235                 240

His Asp Ile Ala Leu Leu Arg Leu His Gln Pro Val Val Leu Thr Asp
                245                 250                 255

His Val Val Pro Leu Cys Leu Pro Glu Arg Thr Phe Ser Glu Arg Thr
            260                 265                 270

Leu Ala Phe Val Arg Phe Ser Leu Val Ser Gly Trp Gly Gln Leu Leu
        275                 280                 285

Asp Arg Gly Ala Thr Ala Leu Glu Leu Met Val Leu Asn Val Pro Arg
    290                 295                 300

Leu Met Thr Gln Asp Cys Leu Gln Gln Ser Arg Lys Val Gly Asp Ser

305                 310                 315                 320

Pro Asn Ile Thr Glu Tyr Met Phe Cys Ala Gly Tyr Ser Asp Gly Ser
                325                 330                 335

Lys Asp Ser Cys Lys Gly Asp Ser Gly Gly Pro His Ala Thr His Tyr
            340                 345                 350

Arg Gly Thr Trp Tyr Leu Thr Gly Ile Val Ser Trp Gly Gln Gly Cys
        355                 360                 365

Ala Thr Val Gly His Phe Gly Val Tyr Thr Arg Val Ser Gln Tyr Ile
    370                 375                 380

Glu Trp Leu Gln Lys Leu Met Arg Ser Glu Pro Arg Pro Gly Val Leu
385                 390                 395                 400

Leu Arg Ala Pro Phe Pro
                405

<210>  6

<211>  406

<212>  PRT

<213>  Homo sapiens

<220>

<221>  Mature Factor VII protein including I140S SNP

<222>  (1)..(406)

<223>

<400>  6

Ala Asn Ala Phe Leu Glu Glu Leu Arg Pro Gly Ser Leu Glu Arg Glu
1               5                   10                  15

Cys Lys Glu Glu Gln Cys Ser Phe Glu Glu Ala Arg Glu Ile Phe Lys
            20                  25                  30

Asp Ala Glu Arg Thr Lys Leu Phe Trp Ile Ser Tyr Ser Asp Gly Asp
        35                  40                  45

Gln Cys Ala Ser Ser Pro Cys Gln Asn Gly Gly Ser Cys Lys Asp Gln
    50                  55                  60

Leu Gln Ser Tyr Ile Cys Phe Cys Leu Pro Ala Phe Glu Gly Arg Asn
65                  70                  75                  80

Cys Glu Thr His Lys Asp Asp Gln Leu Ile Cys Val Asn Glu Asn Gly
                85                  90                  95

Gly Cys Glu Gln Tyr Cys Ser Asp His Thr Gly Thr Lys Arg Ser Cys
            100                 105                 110

Arg Cys His Glu Gly Tyr Ser Leu Leu Ala Asp Gly Val Ser Cys Thr
        115                 120                 125

Pro Thr Val Glu Tyr Pro Cys Gly Lys Ile Pro Ser Leu Glu Lys Arg
    130                 135                 140

Asn Ala Ser Lys Pro Gln Gly Arg Ile Val Gly Gly Lys Val Cys Pro
145                 150                 155                 160

Lys Gly Glu Cys Pro Trp Gln Val Leu Leu Leu Val Asn Gly Ala Gln
                165                 170                 175

Leu Cys Gly Gly Thr Leu Ile Asn Thr Ile Trp Val Val Ser Ala Ala
            180                 185                 190

His Cys Phe Asp Lys Ile Lys Asn Trp Arg Asn Leu Ile Ala Val Leu
        195                 200                 205

Gly Glu His Asp Leu Ser Glu His Asp Gly Asp Glu Gln Ser Arg Arg
    210                 215                 220

Val Ala Gln Val Ile Ile Pro Ser Thr Tyr Val Pro Gly Thr Thr Asn
225                 230                 235                 240

His Asp Ile Ala Leu Leu Arg Leu His Gln Pro Val Val Leu Thr Asp
                245                 250                 255

His Val Val Pro Leu Cys Leu Pro Glu Arg Thr Phe Ser Glu Arg Thr
            260                 265                 270

Leu Ala Phe Val Arg Phe Ser Leu Val Ser Gly Trp Gly Gln Leu Leu
        275                 280                 285

Asp Arg Gly Ala Thr Ala Leu Glu Leu Met Val Leu Asn Val Pro Arg
    290                 295                 300

Leu Met Thr Gln Asp Cys Leu Gln Gln Ser Arg Lys Val Gly Asp Ser
305                 310                 315                 320

Pro Asn Ile Thr Glu Tyr Met Phe Cys Ala Gly Tyr Ser Asp Gly Ser
                325                 330                 335

Lys Asp Ser Cys Lys Gly Asp Ser Gly Gly Pro His Ala Thr His Tyr
            340                 345                 350

Arg Gly Thr Trp Tyr Leu Thr Gly Ile Val Ser Trp Gly Gln Gly Cys
        355                 360                 365

Ala Thr Val Gly His Phe Gly Val Tyr Thr Arg Val Ser Gln Tyr Ile
    370                 375                 380

Glu Trp Leu Gln Lys Leu Met Arg Ser Glu Pro Arg Pro Gly Val Leu
385                 390                 395                 400

Leu Arg Ala Pro Phe Pro

                405

<210>  7

<211>  406

<212>  PRT

<213>  Homo sapiens


<220>

<221>  Mature Factor VII protein including E215K SNP

<222>  (1)..(406)

<223>


<400>  7

Ala Asn Ala Phe Leu Glu Glu Leu Arg Pro Gly Ser Leu Glu Arg Glu
1               5                   10                  15

Cys Lys Glu Glu Gln Cys Ser Phe Glu Glu Ala Arg Glu Ile Phe Lys
            20                  25                  30

Asp Ala Glu Arg Thr Lys Leu Phe Trp Ile Ser Tyr Ser Asp Gly Asp
        35                  40                  45

Gln Cys Ala Ser Ser Pro Cys Gln Asn Gly Gly Ser Cys Lys Asp Gln
    50                  55                  60

Leu Gln Ser Tyr Ile Cys Phe Cys Leu Pro Ala Phe Glu Gly Arg Asn
65                  70                  75                  80

Cys Glu Thr His Lys Asp Asp Gln Leu Ile Cys Val Asn Glu Asn Gly
                85                  90                  95

Gly Cys Glu Gln Tyr Cys Ser Asp His Thr Gly Thr Lys Arg Ser Cys
            100                 105                 110

Arg Cys His Glu Gly Tyr Ser Leu Leu Ala Asp Gly Val Ser Cys Thr
        115                 120                 125

Pro Thr Val Glu Tyr Pro Cys Gly Lys Ile Pro Ile Leu Glu Lys Arg
    130                 135                 140

Asn Ala Ser Lys Pro Gln Gly Arg Ile Val Gly Gly Lys Val Cys Pro
145                 150                 155                 160

Lys Gly Glu Cys Pro Trp Gln Val Leu Leu Leu Val Asn Gly Ala Gln
                165                 170                 175

Leu Cys Gly Gly Thr Leu Ile Asn Thr Ile Trp Val Val Ser Ala Ala
            180                 185                 190

His Cys Phe Asp Lys Ile Lys Asn Trp Arg Asn Leu Ile Ala Val Leu
        195                 200                 205

Gly Glu His Asp Leu Ser Lys His Asp Gly Asp Glu Gln Ser Arg Arg
    210                 215                 220

Val Ala Gln Val Ile Ile Pro Ser Thr Tyr Val Pro Gly Thr Thr Asn
225                 230                 235                 240

His Asp Ile Ala Leu Leu Arg Leu His Gln Pro Val Val Leu Thr Asp
                245                 250                 255

His Val Val Pro Leu Cys Leu Pro Glu Arg Thr Phe Ser Glu Arg Thr

            260                 265                 270

Leu Ala Phe Val Arg Phe Ser Leu Val Ser Gly Trp Gly Gln Leu Leu
        275                 280                 285

Asp Arg Gly Ala Thr Ala Leu Glu Leu Met Val Leu Asn Val Pro Arg
    290                 295                 300

Leu Met Thr Gln Asp Cys Leu Gln Gln Ser Arg Lys Val Gly Asp Ser
305                 310                 315                 320

Pro Asn Ile Thr Glu Tyr Met Phe Cys Ala Gly Tyr Ser Asp Gly Ser
                325                 330                 335

Lys Asp Ser Cys Lys Gly Asp Ser Gly Gly Pro His Ala Thr His Tyr
            340                 345                 350

Arg Gly Thr Trp Tyr Leu Thr Gly Ile Val Ser Trp Gly Gln Gly Cys
        355                 360                 365

Ala Thr Val Gly His Phe Gly Val Tyr Thr Arg Val Ser Gln Tyr Ile
    370                 375                 380

Glu Trp Leu Gln Lys Leu Met Arg Ser Glu Pro Arg Pro Gly Val Leu
385                 390                 395                 400

Leu Arg Ala Pro Phe Pro
                405

<210> 8
<211> 406

<212> PRT

<213> Homo sapiens

<220>

<221> Mature Factor VII protein
including R224Q SNP
<222> (1)..(406)

<223>

<400> 8

Ala Asn Ala Phe Leu Glu Glu Leu Arg Pro Gly Ser Leu Glu Arg Glu
1               5                   10                  15

Cys Lys Glu Glu Gln Cys Ser Phe Glu Glu Ala Arg Glu Ile Phe Lys
            20                  25                  30

Asp Ala Glu Arg Thr Lys Leu Phe Trp Ile Ser Tyr Ser Asp Gly Asp
        35                  40                  45

Gln Cys Ala Ser Ser Pro Cys Gln Asn Gly Gly Ser Cys Lys Asp Gln
    50                  55                  60

Leu Gln Ser Tyr Ile Cys Phe Cys Leu Pro Ala Phe Glu Gly Arg Asn
65                  70                  75                  80

Cys Glu Thr His Lys Asp Asp Gln Leu Ile Cys Val Asn Glu Asn Gly
                85                  90                  95

Gly Cys Glu Gln Tyr Cys Ser Asp His Thr Gly Thr Lys Arg Ser Cys
            100                 105                 110

Arg Cys His Glu Gly Tyr Ser Leu Leu Ala Asp Gly Val Ser Cys Thr
        115                 120                 125

Pro Thr Val Glu Tyr Pro Cys Gly Lys Ile Pro Ile Leu Glu Lys Arg
    130                 135                 140

Asn Ala Ser Lys Pro Gln Gly Arg Ile Val Gly Gly Lys Val Cys Pro
145                 150                 155                 160

Lys Gly Glu Cys Pro Trp Gln Val Leu Leu Leu Val Asn Gly Ala Gln
                165                 170                 175

Leu Cys Gly Gly Thr Leu Ile Asn Thr Ile Trp Val Val Ser Ala Ala
            180                 185                 190

His Cys Phe Asp Lys Ile Lys Asn Trp Arg Asn Leu Ile Ala Val Leu
        195                 200                 205

Gly Glu His Asp Leu Ser Glu His Asp Gly Asp Glu Gln Ser Arg Gln
    210                 215                 220

Val Ala Gln Val Ile Ile Pro Ser Thr Tyr Val Pro Gly Thr Thr Asn
225                 230                 235                 240

His Asp Ile Ala Leu Leu Arg Leu His Gln Pro Val Val Leu Thr Asp

                245                 250                 255

His Val Val Pro Leu Cys Leu Pro Glu Arg Thr Phe Ser Glu Arg Thr
            260                 265                 270

Leu Ala Phe Val Arg Phe Ser Leu Val Ser Gly Trp Gly Gln Leu Leu
        275                 280                 285

Asp Arg Gly Ala Thr Ala Leu Glu Leu Met Val Leu Asn Val Pro Arg
    290                 295                 300

Leu Met Thr Gln Asp Cys Leu Gln Gln Ser Arg Lys Val Gly Asp Ser
305                 310                 315                 320

Pro Asn Ile Thr Glu Tyr Met Phe Cys Ala Gly Tyr Ser Asp Gly Ser
                325                 330                 335

Lys Asp Ser Cys Lys Gly Asp Ser Gly Gly Pro His Ala Thr His Tyr
            340                 345                 350

Arg Gly Thr Trp Tyr Leu Thr Gly Ile Val Ser Trp Gly Gln Gly Cys
        355                 360                 365

Ala Thr Val Gly His Phe Gly Val Tyr Thr Arg Val Ser Gln Tyr Ile
    370                 375                 380

Glu Trp Leu Gln Lys Leu Met Arg Ser Glu Pro Arg Pro Gly Val Leu
385                 390                 395                 400

Leu Arg Ala Pro Phe Pro
                405

<210> 9
<211> 406

<212> PRT

<213> Homo sapiens

<220>

<221> Mature Factor VII protein
including A294S SNP
<222> (1)..(406)

<223>

<400> 9

Ala Asn Ala Phe Leu Glu Glu Leu Arg Pro Gly Ser Leu Glu Arg Glu
1               5                   10                  15

Cys Lys Glu Glu Gln Cys Ser Phe Glu Glu Ala Arg Glu Ile Phe Lys
            20                  25                  30

Asp Ala Glu Arg Thr Lys Leu Phe Trp Ile Ser Tyr Ser Asp Gly Asp
        35                  40                  45

Gln Cys Ala Ser Ser Pro Cys Gln Asn Gly Gly Ser Cys Lys Asp Gln
    50                  55                  60

Leu Gln Ser Tyr Ile Cys Phe Cys Leu Pro Ala Phe Glu Gly Arg Asn
65                  70                  75                  80

Cys Glu Thr His Lys Asp Asp Gln Leu Ile Cys Val Asn Glu Asn Gly
                85                  90                  95

Gly Cys Glu Gln Tyr Cys Ser Asp His Thr Gly Thr Lys Arg Ser Cys
            100                 105                 110

Arg Cys His Glu Gly Tyr Ser Leu Leu Ala Asp Gly Val Ser Cys Thr
        115                 120                 125

Pro Thr Val Glu Tyr Pro Cys Gly Lys Ile Pro Ile Leu Glu Lys Arg
    130                 135                 140

Asn Ala Ser Lys Pro Gln Gly Arg Ile Val Gly Gly Lys Val Cys Pro
145                 150                 155                 160

Lys Gly Glu Cys Pro Trp Gln Val Leu Leu Leu Val Asn Gly Ala Gln
                165                 170                 175

Leu Cys Gly Gly Thr Leu Ile Asn Thr Ile Trp Val Val Ser Ala Ala
            180                 185                 190

His Cys Phe Asp Lys Ile Lys Asn Trp Arg Asn Leu Ile Ala Val Leu
        195                 200                 205

Gly Glu His Asp Leu Ser Glu His Asp Gly Asp Glu Gln Ser Arg Arg
    210                 215                 220

Val Ala Gln Val Ile Ile Pro Ser Thr Tyr Val Pro Gly Thr Thr Asn
225                 230                 235                 240

His Asp Ile Ala Leu Leu Arg Leu His Gln Pro Val Val Leu Thr Asp
                245                 250                 255

His Val Val Pro Leu Cys Leu Pro Glu Arg Thr Phe Ser Glu Arg Thr
            260                 265                 270

Leu Ala Phe Val Arg Phe Ser Leu Val Ser Gly Trp Gly Gln Leu Leu
        275                 280                 285

Asp Arg Gly Ala Thr Ser Leu Glu Leu Met Val Leu Asn Val Pro Arg
    290                 295                 300

Leu Met Thr Gln Asp Cys Leu Gln Gln Ser Arg Lys Val Gly Asp Ser
305                 310                 315                 320

Pro Asn Ile Thr Glu Tyr Met Phe Cys Ala Gly Tyr Ser Asp Gly Ser
                325                 330                 335

Lys Asp Ser Cys Lys Gly Asp Ser Gly Gly Pro His Ala Thr His Tyr
            340                 345                 350

Arg Gly Thr Trp Tyr Leu Thr Gly Ile Val Ser Trp Gly Gln Gly Cys
        355                 360                 365

Ala Thr Val Gly His Phe Gly Val Tyr Thr Arg Val Ser Gln Tyr Ile
    370                 375                 380

Glu Trp Leu Gln Lys Leu Met Arg Ser Glu Pro Arg Pro Gly Val Leu
385                 390                 395                 400

Leu Arg Ala Pro Phe Pro
                405

<210>  10

<211>  406

<212>  PRT

<213>  Homo sapiens

<220>

<221>  Mature Factor VII protein
 including T324M SNP
<222>  (1)..(406)

<223>

<400>  10

Ala Asn Ala Phe Leu Glu Glu Leu Arg Pro Gly Ser Leu Glu Arg Glu
1               5                   10                  15

Cys Lys Glu Glu Gln Cys Ser Phe Glu Glu Ala Arg Glu Ile Phe Lys
            20                  25                  30

Asp Ala Glu Arg Thr Lys Leu Phe Trp Ile Ser Tyr Ser Asp Gly Asp
        35                  40                  45

Gln Cys Ala Ser Ser Pro Cys Gln Asn Gly Gly Ser Cys Lys Asp Gln
    50                  55                  60

Leu Gln Ser Tyr Ile Cys Phe Cys Leu Pro Ala Phe Glu Gly Arg Asn
65                  70                  75                  80

Cys Glu Thr His Lys Asp Asp Gln Leu Ile Cys Val Asn Glu Asn Gly
                85                  90                  95

Gly Cys Glu Gln Tyr Cys Ser Asp His Thr Gly Thr Lys Arg Ser Cys
            100                 105                 110

Arg Cys His Glu Gly Tyr Ser Leu Leu Ala Asp Gly Val Ser Cys Thr
        115                 120                 125

Pro Thr Val Glu Tyr Pro Cys Gly Lys Ile Pro Ile Leu Glu Lys Arg
    130                 135                 140

Asn Ala Ser Lys Pro Gln Gly Arg Ile Val Gly Gly Lys Val Cys Pro
145                 150                 155                 160

Lys Gly Glu Cys Pro Trp Gln Val Leu Leu Leu Val Asn Gly Ala Gln
                165                 170                 175

Leu Cys Gly Gly Thr Leu Ile Asn Thr Ile Trp Val Val Ser Ala Ala
            180                 185                 190

His Cys Phe Asp Lys Ile Lys Asn Trp Arg Asn Leu Ile Ala Val Leu
        195                 200                 205

Gly Glu His Asp Leu Ser Glu His Asp Gly Asp Glu Gln Ser Arg Arg
    210                 215                 220

Val Ala Gln Val Ile Ile Pro Ser Thr Tyr Val Pro Gly Thr Thr Asn
225                 230                 235                 240

His Asp Ile Ala Leu Leu Arg Leu His Gln Pro Val Val Leu Thr Asp
                245                 250                 255

His Val Val Pro Leu Cys Leu Pro Glu Arg Thr Phe Ser Glu Arg Thr
            260                 265                 270

Leu Ala Phe Val Arg Phe Ser Leu Val Ser Gly Trp Gly Gln Leu Leu
        275                 280                 285

Asp Arg Gly Ala Thr Ala Leu Glu Leu Met Val Leu Asn Val Pro Arg
    290                 295                 300

Leu Met Thr Gln Asp Cys Leu Gln Gln Ser Arg Lys Val Gly Asp Ser
305                 310                 315                 320

Pro Asn Ile Met Glu Tyr Met Phe Cys Ala Gly Tyr Ser Asp Gly Ser
                325                 330                 335

Lys Asp Ser Cys Lys Gly Asp Ser Gly Gly Pro His Ala Thr His Tyr
            340                 345                 350

Arg Gly Thr Trp Tyr Leu Thr Gly Ile Val Ser Trp Gly Gln Gly Cys
        355                 360                 365

Ala Thr Val Gly His Phe Gly Val Tyr Thr Arg Val Ser Gln Tyr Ile
    370                 375                 380

Glu Trp Leu Gln Lys Leu Met Arg Ser Glu Pro Arg Pro Gly Val Leu
385                 390                 395                 400

Leu Arg Ala Pro Phe Pro
                405

## Description

The present invention relates to new polynucleotides deriving from the nucleotide sequence of the Factor VII (FVII) gene comprising new natural SNPs, new polypeptides encoded by said polynucleotides, as well as their therapeutic uses.

Blood coagulation is a process consisting of a complex interaction of various blood factors that eventually results in a fibrin clot. In general, the factors participating in blood coagulation are proenzymes or zymogens, i.e. enzymatically inactive proteins that are converted into an active form by the action of an activator.

Factor VII (FVII) is one of the essential factors for the initiation of blood coagulation via the tissue factor (TF) pathway. Factor VII is a vitamin K-dependent plasma serine protease synthesized in the liver and secreted into the blood as a single-chain glycoprotein with a molecular weight of 53 kDa (Broze & Majerus (1980) J. Biol. Chem., 255:1242-1247). FVII under its zymogen form is catalytically activated upon binding to its cell surface receptor and co-factor tissue factor (TF) into an activated form FVIIa by proteolytic cleavage at a single site, R152-I153; resulting in two chains of 152 and 254 amino acids, respectively, linked by a single disulfide bridge (Pike et al., (1999) Proc. Natl. Acad. Sci., 96: 8925-8930).

The gene coding for human FVII (*hFVII*) has been mapped to chromosome 13 at q34-qter 9. It contains nine exons and spans 12.8 Kb. The gene organization and protein structure of FVII are similar to those of other vitamin K-dependent procoagulant proteins, with exons 1 a and 1b encoding for signal sequence; exon 2 the propeptide and Gla domain; exon 3 a short hydrophobic region; exons 4 and 5 the epidermal growth factor-like domains; and exon 6 through 8 the serine protease catalytic domain (Yoshitake et al., (1985) Biochemistry, 24: 3736-3750).

In case of injury, Tissue factor (TF) is expressed at the site of damaged endothelial cells. TF forms a complex with FVIIa (FVIIa complex), which is able to convert both factor IX and factor X into their activated forms, followed by reactions leading to rapid thrombin production and fibrin formation.

Severe reduction in plasma FVII activity usually leads to bleeding problems in human patients although the phenotype is heterogeneous. Complete absence of circulating FVII appears to be lethal due to traumatic post-natal bleeding (Cooper et al., (1997) Thromb. Haemostasis, 78: 151-160). In addition, a recent report on FVII knock-out mice indicates poor survival of neonates, with the majority dying within 24 hours from severe bleeding and virtually no survival beyond 21 days (Rosen et al., (1997) Nature, 390: 290-293).

WO 96/12800 describes inactivation of FVlla by a serine proteinase inhibitor. This inactivated form is capable of competing with wild-type FVII or FVIIa for binding to tissue factor and inhibiting clotting activity. Meanwhile, the inactivated form of FVIIa is suggested to be used for treatment of patients being in hypercoagulable states, such as patients with sepsis, in risk of myocardial infarction or of thrombotic stroke.

In connection with treatment of uncontrolled bleedings, such as trauma, it is believed that factor VIIa is capable of activating factor X to factor Xa without binding to tissue factor, and this activation reaction is believed to occur primarily on activated blood platelets (Hedner et al., (2000) Blood Coagulation & Fibrinolysis, 11: 107-111). However, FVIIa has a low activity towards factor X in the absence of tissue factor and, consequently, treatment of uncontrolled bleeding, for example in trauma patients, requires relatively high and multiple doses of FVIIa. Therefore, in order to treat uncontrolled bleedings more efficiently (to minimize blood loss) there is need for improved FVIIa molecules, which possess a high activity toward factor X in the absence of tissue factor. Such improved FVIIa molecules will exhibit a lowered clotting time (or faster action) as compared to FVIIa when administered in connection with uncontrolled bleedings.

Recombinant factor VIIa (rFVIIa) manufactured by Novo Nordisk (known under the trademark Novoseven®) has been approved to treat bleeding episodes in the following groups of patients :
- patients with congenital haemophilia A or B with inhibitors or factor VII deficiency;
- patients suffering from a Glanzmann's thrombasthenia with antibodies anti-GP llb-lla and/or anti-HLA ;
- patients with acquired haemophilia (Unlike classic haemophilia, acquired haemophilia is not an inherited condition). The onset usually occurs well into adulthood and is often associated with other conditions such as autoimmune disorders and cancer or with the use of certain drugs. Bleeding episodes in people with acquired haemophilia occur mostly in the skin and soft tissue. They are a result of the body producing antibodies that attack essential clotting factors VIII and IX, which hinder coagulation);
- in the prevention of bleeding in surgical interventions or invasive procedures in patients with haemophilia A or B.

Since the approval of recombinant factor VIIa from Novo Nordisk, it has seen increasing use in patients without haemophilia for the control of refractory bleeding in various clinical situations (A study on Consesus Recomendations for the Off-Label Use of Recombinant Human Factor Vlla NovoSeven® Therapy, published in P&T (2005) 30(11): 644-658). This study found that the use of rFVIIa is appropriate in limited circumstances including cardiac, thoracic aortic, or spinal surgery, hepatic rejection, hysterectomy, postpartum bleeding, severe multiple trauma and non traumatic intracranial bleeding.

Extensive mutational analysis of Factor VII protein have been reported in the literature with respect to TF binding and factor X hydrolysis in view to define which residues of the protein are involved in these interactions (Dickinson, C.D. et al. (1996) Proc. Natl. Acad. Sci., 93:14379-14384). In these experiments, only the substitution mutations impairing FVII functionality were considered. These studies revealed that factor TF.VIIa interacts with its macromolecular substrate through an extended interface involving a large array of residues

WO 03/093465 relates to novel polypeptide variants of recombinant Factor VII or VIIa polypeptides, where said variants comprise an amino acid substitution in position 10 and 32 within the Gla domain and where said variants further comprise a sugar moiety covalently attached to an introduced *in vivo* N-glycosylation site. The modifications performed in the Gla domain of the wild type protein are of nature that an increased phospholipid membrane binding affinity is achieved, so that the resulting molecule has an improved capability to activate FX to FXa in order to form a stronger clot. However, these artificial mutants only concern modifications of the Gla domain of the protein, which interacts with FX.

In relation with genetically inherited blood coagulation disorders, such as those encountered in hemophiliac persons, frequent gene mutations have been identified impairing Factor VII functionality (Toso, R. et al. (2002) Biochem. J., 363: 411- 416) (Borensztajn, K. et al. (2003) Blood, 102(2):561-563). However, it has not been reported in these studies natural human mutations found in the human natural gene of Factor VII having a positive impact on Factor VII functionality, i.e. conducting to coagulation faster than the wild type factor VII and/or to longer activity (half-life) than the wild type factor VII.

The present inventors have investigated allelic copies of FVII human gene in human population not especially concerned by inherited blood disorders. This approach has unexpectedly led to identify natural functionally single nucleotide polymorphisms (SNPs) in the exons of the gene encoding FVII protein.

Six of these functionally SNPs are considered as positively acting upon Factor VII protein functionality, in particular in view of the molecular bio-informatics predictive structure analysis of the Factor VII variants containing them.

The polynucleotides comprising at least one of these 6 exonic SNPs and their resulting polypeptides are the object of the present invention, as well as their use for diagnose, treat or prevent blood coagulation related disorders, bleedings, hemorrhages or other pathologies such as those mentioned previously, in particular by improving the properties of natural or recombinant Factor VII proteins.

### BRIEF SUMMARY OF THE INVENTION

The invention has as its first object new polynucleotides that differ from the nucleotide sequence of the reference wild-type FVII gene in that they comprise one or several selected SNPs (Single Nucleotide Polymorphism).

The nucleotide sequence SEQ ID NO. 1 of the human reference wild-type FVII gene is composed of 14241 nucleotides and comprises a coding sequence of 1401 nucleotides beginning at nucleotide 52 (start codon) and ending at nucleotide 13218 (stop codon) in the human reference wild-type FVII gene.

The FVII gene is composed of 9 exons placed at the following positions on the nucleotide sequence SEQ ID NO. 1:
Exon 1: from nucleotide 1 to nucleotide 115 (Start codon at position 1)
Exon 2: from nucleotide 1055 to nucleotide 1120
Exon 3: from nucleotide 4900 to nucleotide 5060
Exon 4: from nucleotide 7962 to nucleotide 7986
Exon 5: from nucleotide 8057 to nucleotide 8170
Exon 6: from nucleotide 9870 to nucleotide 10010
Exon 7: from nucleotide 10976 to nucleotide 11085
Exon 8: from nucleotide 11683 to nucleotide 11806
Exon 9: from nucleotide 12623 to nucleotide 14891 (Stop codon at position 13218)

The applicant has identified and selected 6 SNPs in the nucleotide sequence of the reference wild-type FVII gene. These SNPs are the following ones: c9951t, t11004g, g12640a, g12668a, g12877t, c12968t for which the numbering corresponds to the positioning of the SNP relative to the numbering of the nucleotide sequence SEQ ID N0.1. These SNPs are located in the exons of the wild type FVII gene, they thus take part the coding sequence of FVII represented by SEQ ID NO.2 under the respective SNPs: c512t, t599g, g823a, g851a, g1060t, c1151t, for which the numbering corresponds to the positioning of the SNP relative to said nucleotide sequence SEQ ID NO. 2.

The letters a, t, c, and g correspond respectively to the nitrogenous bases adenine, thymine, cytosine and guanine. The first letter corresponds to the wild-type nucleotide, whereas the last letter corresponds to the mutated nucleotide.

Thus, for example, the SNP c9951t (c512t) mentioned above corresponds to a mutation of the nucleotide c (cytosine) into t (thymine) at position 9951 of the reference nucleotide sequence SEQ ID NO. 1 encoding wild-type FVII (position 512 in the reference coding sequence SEQ ID NO. 2 of wild-type FVII).

The nucleotide SNPs according to the invention have been identified by sequencing FVII gene fragments isolated and amplified using PCR amplification, from the genomes of 384 individuals randomly sampled in the worldwide human population.

The reference wild-type FVII gene codes for an immature protein of 466 amino acids. The amino acid sequence of said immature FVII protein is represented by the amino acid sequence SEQ ID NO. 3. The immature protein is converted to the 406 amino acid mature form of the protein by cleavage of both the signal peptide and the pro-peptide that includes the first 60 amino acids. The amino acid sequence of the mature form of the FVII protein is represented by SEQ ID NO.4.

The above nucleotide SNPs cause translational modifications at the level of the FVII amino acid sequence (peptide SNPs) according to the invention, which are noted here below:
c9951t (c512t) → S111F
t11004g (t599g) → I140S
g12640a (g823a) → E215K
g12668a (g851a) → R224Q
g12877t (g1060t) → A294S
c12968t (c1151t) → T324M

The SNP S111 F corresponds to a mutation of the Serine (S) into Phenylalanine (F) at position 111 in the mature protein of the FVII (SEQ ID NO. 4), which corresponds to position 171 in the immature amino acid sequence (SEQ ID NO.3).

The SNP I140S corresponds to a mutation of the Isoleucine (I) into Serine (S) at position 140 in the mature protein of the FVII (SEQ ID NO. 4), which corresponds to position 200 in the immature amino acid sequence (SEQ ID NO.3).

The SNP E215K corresponds to a mutation of the Glutamic acid (E) into Lysine (K) at position 215 in the mature protein of the FVII (SEQ ID NO. 4), which corresponds to position 284 in the immature sequence (SEQ ID NO.3).

The SNP R224Q corresponds to a mutation of the Arginine (R) into Glutamine (Q) at position 224 in the mature protein of the FVII (SEQ ID NO. 4), which corresponds to position 284 in the immature sequence (SEQ ID NO.3).

The SNP A294S corresponds to a mutation of the Alanine (A) into Serine (S) at position 294 in the mature protein of the FVII (SEQ ID NO. 4), which corresponds to position 354 in the immature sequence (SEQ ID NO.3).

The SNP T324M corresponds to a mutation of the Threonine (T) into Methionine (M) at position 324 in the mature protein of the FVII (SEQ ID NO. 4), which corresponds to position 384 in the immature sequence (SEQ ID NO.3).

The mature FVII protein amino acid sequence including S111 F (i.e. where F is found at position 111) corresponds to SEQ ID NO.5.

The mature FVII protein amino acid sequence including 1140S (i.e. where S is found at position 140) corresponds to SEQ ID NO.6.

The mature FVII protein amino acid sequence including E215K (i.e. where K is found at position 215) corresponds to SEQ ID NO.7.

The mature FVII protein amino acid sequence including R224Q (i.e. where Q is found at position 224) corresponds to SEQ ID NO.8.

The mature FVII protein amino acid sequence including A294S (i.e. where S is found at position 294) corresponds to SEQ ID NO.9.

The mature FVII protein amino acid sequence including T324M (i.e. where M is found at position 324) corresponds to SEQ ID N0.10.

The SNPs S111F, 1140S, E215K, R224Q, A294S and T324M cause modifications of the spatial conformation of the wild type FVII polypeptide as observed by computational molecular modelling, such as MODELER/Accelrys (homology modelling), DELPHI/Accelrys (electrostatic potentials), and/or molecular simulation (using force field to determine minimum energy conformations as well as dynamic trajectories of molecular systems) such as TINKER/FFE package (Ren, P. and Ponder J. W. (2003) J. Phys. Chem. B, 107:5933-5947).

Amino acid residue at position 111 in the mature FVII protein is placed in the EGF2 domain of said protein in a beta-strand. The replacement of Serine, which is a polar and tiny residue, at position 111, by Phenylalanine, which is a bulky and aromatic residue, provokes a polar to hydrophobic inversion of amino acid property. More, the residue volume dramatically increases. This modifies the residue features and thus affects the local structure of the protein. Moreover, this amino acid is close to residues involved in the interaction with tissue factor (TF). It is placed just before Cys112 involved in a disulfide bridge and after the highly conserved Arg110 residue that is structurally important. SNP S111F is thus plays a structural role in Factor VII and in the interaction with tissue factor (TF).

Amino acid residue at position 140 in the mature FVII protein is placed in the connecting region between the EGF2 domain and the peptidase domain and close to the R152-Ile153 cleavage site. This amino acid is located in a loop region. The replacement of isoleucine, which is a hydrophobic amino acid, by serine, which is a polar and tiny residue, causes an hydrophobic to polar inversion of amino acid property. Moreover, this amino acid residue is located close to the Asn145 glycosylation site, which residue is important for the cleavage (FVII activation) of Arg152. Apparition of a polar residue (Serine) can increase flexibility of the connecting region and 1140S SNP thus impact positively the activation of factor VII.

Amino acid residue at position 215 in the mature FVII protein is placed in the peptidase domain in the loop region. The replacement of glutamic acid, which is a negatively-charged residue, at position 215, by lysine, which is a positively-charged residue, causes an inversion of charge. The position of this residue is close to the catalytic cleft. According to Kumar et al. (1993, Eur. J. Biochem. 217(2):509-518), the region including Glu215 is reported as forming a FX binding interface with FVII. E215K SNP should positively impact the proteolytic activity of Factor VII. In addition, due to the high accessibility of the lysine residue to solvent, this should make the mutated Factor VII sensitive to trypsin protease, which lowers its half-life compared to wild-type FVII.

Amino acid residue at position 224 in the FVII mature protein is placed in a beta strand in the peptidase domain. The replacement of arginine at position 224 by Glutamine causes a loss of positive charge, which conducts to render the protein more resistant against trypsin proteolysis. The position of this residue is close to the catalytic cleft. R224Q SNP thus positively impacts the proteolytic activity of Factor VII as well as increases the half-life of Factor VII.

Amino acid residue at position 294 in the FVII mature protein is placed in the peptidase domain. This is a buried residue located in a beta strand. The position of that residue is close to the catalytic cleft. The replacement of alanine at position 294 by serine increases locally the polarity of the protein. According to Kumar et al. (1993, Eur. J. Biochem. 217(2):509-518), the region including Ala294 is reported as forming a FX binding interface with FVII. Thus A294S SNP is also believed to positively impact the proteolytic activity of Factor VII.

Amino acid residue at position 324 in the FVII mature protein is placed in the peptidase domain. This is a partially buried residue located in a loop region. The replacement of threonine at position 324 by methionine, which is a bigger residue, modifies the "322 NIT" N-glycosylation motif and may prevent the glycosylation of Asn322, which impacts protease sensitivity of Factor VII (some sites sensitive to proteases are accessible due to absence of carbohydrate chain), as well as Factor VII activity and binding to tissue factor. T324M SNP thus impacts Factor VII half-life and possibly its activity and/or its interaction with tissue factor (TF).

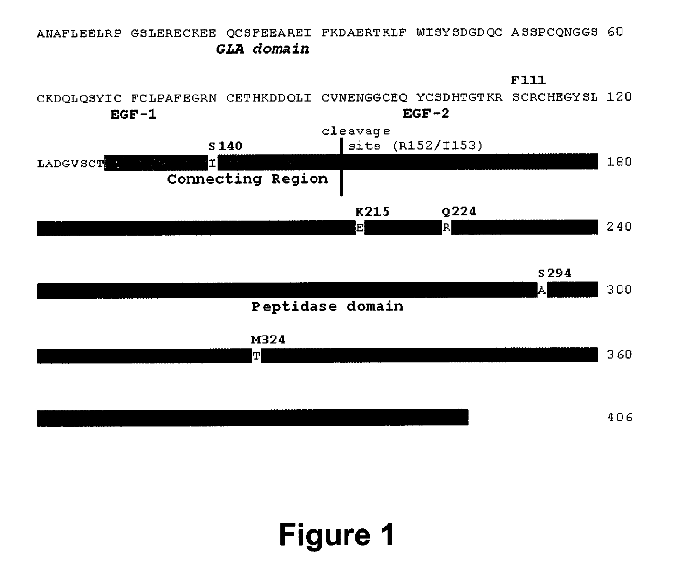

**Figure 1** represents the location of the 6 SNPs according to the invention on the mature FVII protein sequence, with respect to the different domains said protein comprises.

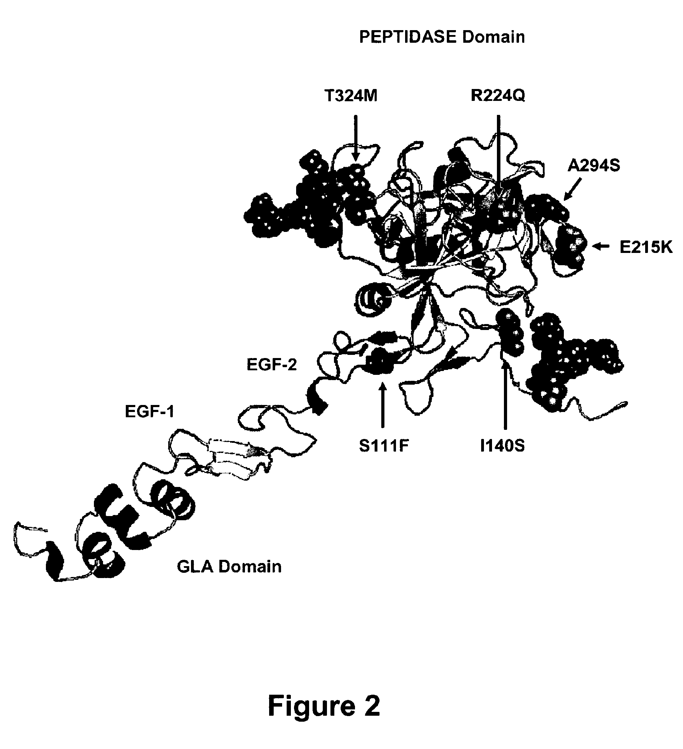

**Figure 2** represents an overview of the SNPs S111F, I140S, E215K, R224Q, A294S and T324M locations in Factor VII 3-D modeled protein structure (image made with Pymol, url : pymol.sourceforge.net).

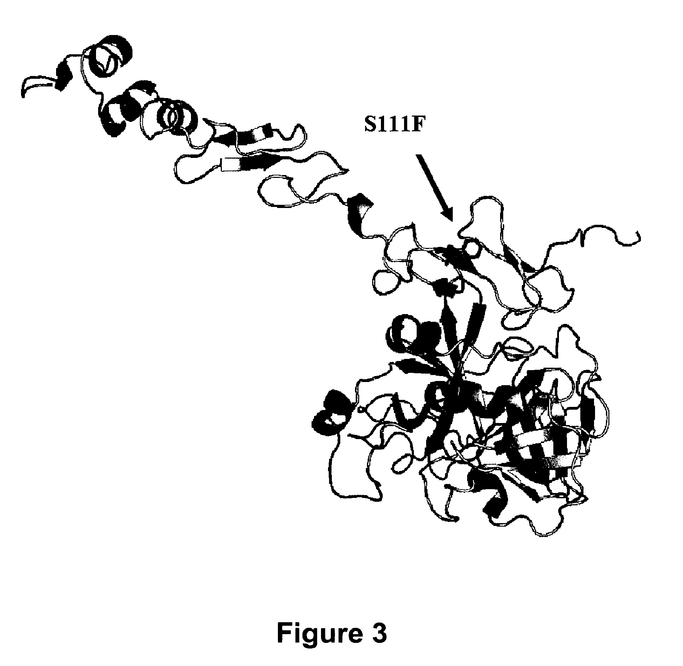

**Figure 3** represents an overview of 3-D Factor VII protein structure including the S111F SNP according to the invention (image made with Pymol, url : pymol.sourceforge.net).

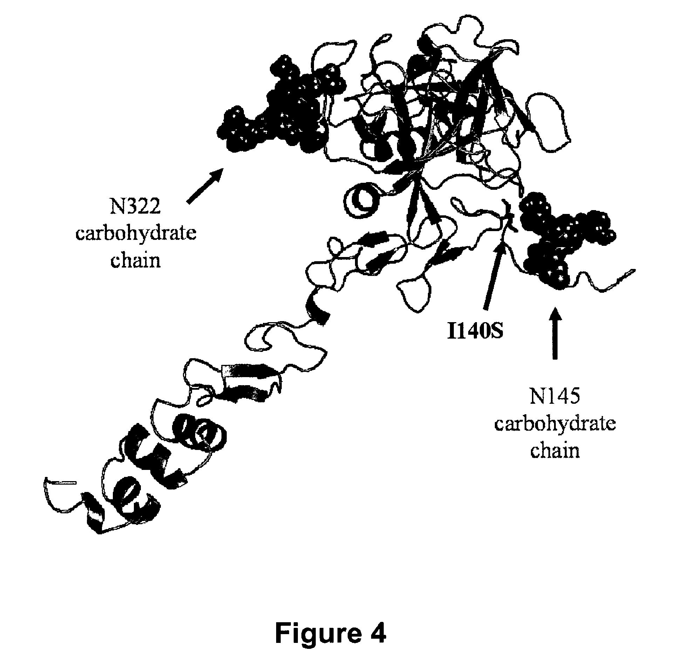

**Figure 4** represents an overview of 3-D Factor VII protein structure including the I140S SNP according to the invention.

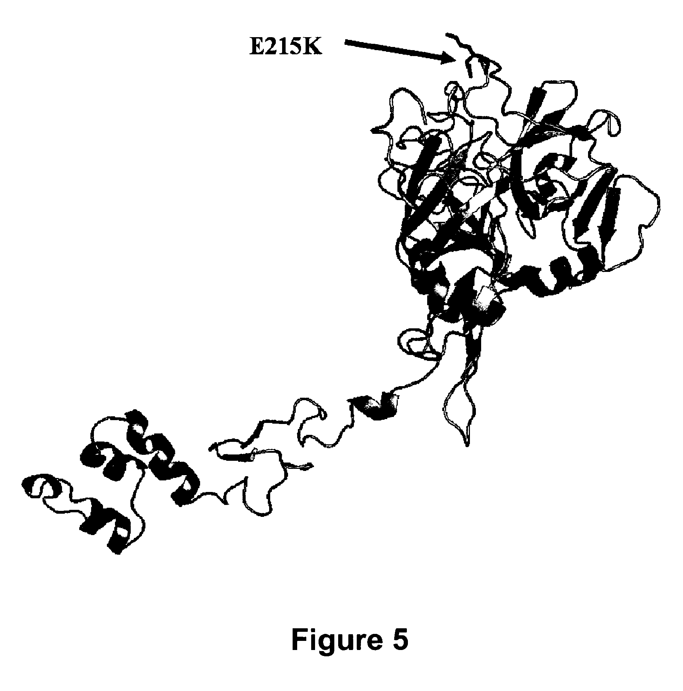

**Figure 5** represents an overview of 3-D Factor VII protein structure including the E215K SNP according to the invention.

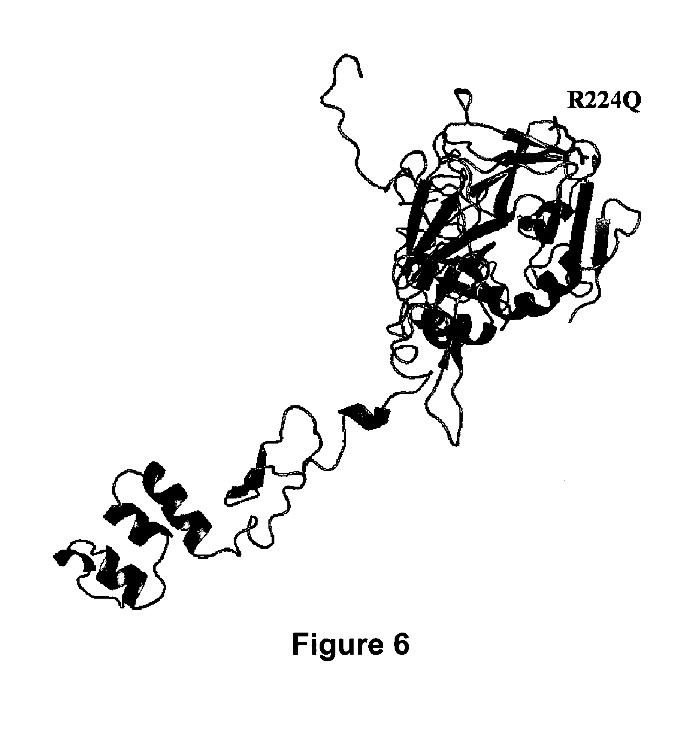

**Figure 6** represents an overview of 3-D Factor VII protein structure including the R224Q SNP according to the invention.

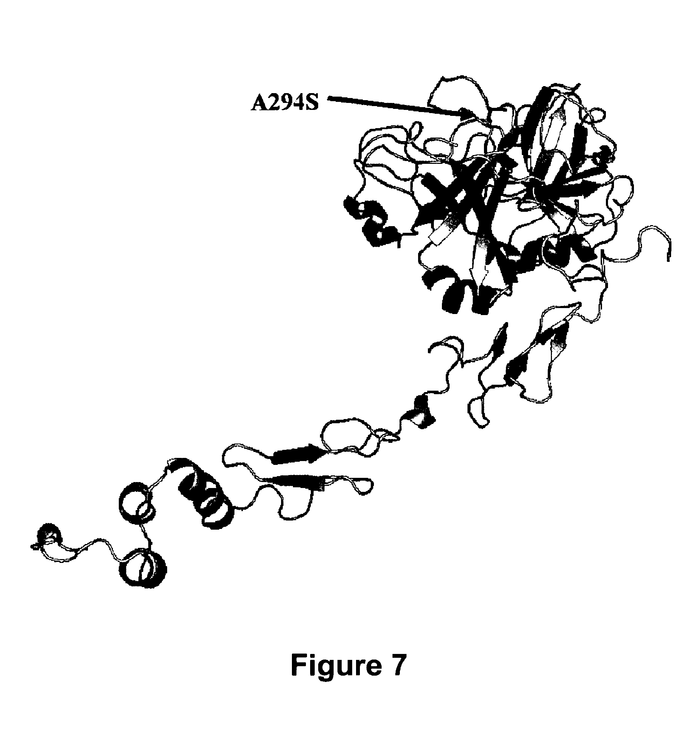

**Figure 7** represents an overview of 3-D Factor VII protein structure including the A294S SNP according to the invention.

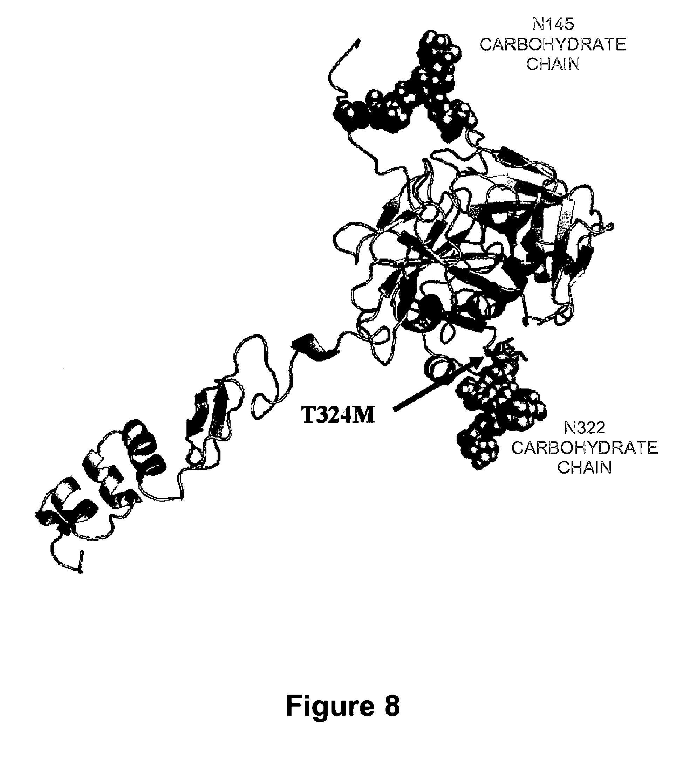

**Figure 8** represents an overview of 3-D Factor VII protein structure including the T324M SNP according to the invention.

### DETAILED DESCRIPTION OF THE INVENTION

### Definitions.

"Nucleotide sequence of the reference wild-type gene" is understood as the nucleotide sequence SEQ ID NO. 1 of the human FVII gene which is accessible in GenBank under Accession number AF466933 and described in Rieder, M.J., et al., 2003, Molecular Biotechnology, University of Washington, 1705 NE Pacific, Seattle, WA 98195, USA.

"Natural wild-type Factor VII protein" is understood as the immature protein encoded by the "coding nucleotide sequence" SEQ ID NO.2 of the reference wild-type FVII gene. The amino acid sequence of the immature FVII protein, which is encoded by said coding nucleotide sequence, corresponds to SEQ ID NO.3. The natural wild-type mature FVII protein also called "FVIIa" corresponds to the peptide sequence SEQ ID NO. 4., which spans amino acids 61 through 466 of SEQ ID NO.3.

"Polynucleotide" is understood as a polyribonucleotide or a polydeoxyribonucleotide that can be a modified or non-modified DNA or RNA. The term polynucleotide includes, for example, a single strand or double strand DNA, a DNA composed of a mixture of one or several single strand region(s) and of one or several double strand region(s), a single strand or double strand RNA and an RNA composed of a mixture of one or several single strand region(s) and of one or several double strand region(s). The term polynucleotide can also include an RNA and/or a DNA including one or several triple strand regions. Polynucleotide is equally understood as the DNAs and RNAs containing one or several bases modified in such a fashion as to have a skeleton modified for reasons of stability or for other reasons. By modified base is understood, for example, the unusual bases such as inosine.

"Polypeptide" is understood as a peptide, an oligopeptide, an oligomer or a protein comprising at least two amino acids joined to each other by a normal or modified peptide bond, such as in the cases of the isosteric peptides, for example.

A polypeptide can be composed of amino acids other than the 20 amino acids defined by the genetic code. A polypeptide can equally be composed of amino acids modified by natural processes, such as post-translational maturation processes or by chemical processes, which are well known to a person skilled in the art. Such modifications are fully detailed in the literature. These modifications can appear anywhere in the polypeptide: in the peptide skeleton, in the amino acid chain or even at the carboxy- or amino-terminal ends.

A polypeptide can be branched following an ubiquitination or be cyclic with or without branching. This type of modification can be the result of natural or synthetic post-translational processes that are well known to a person skilled in the art.

For example, polypeptide modifications is understood to include acetylation, acylation, ADP-ribosylation, amidation, covalent fixation of flavine, covalent fixation of heme, covalent fixation of a nucleotide or of a nucleotide derivative, covalent fixation of a lipid or of a lipidic derivative, the covalent fixation of a phosphatidylinositol, covalent or non-covalent cross-linking, cyclization, disulfide bond formation, demethylation, cysteine formation, pyroglutamate formation, formylation, gamma-carboxylation, glycosylation including pegylation, GPI anchor formation, hydroxylation, iodization, methylation, myristoylation, oxidation, proteolytic processes, phosphorylation, prenylation, racemization, seneloylation, sulfatation, amino acid addition such as arginylation or ubiquitination. Such modifications are fully detailed in the literature (Proteins Structure and Molecular Properties (1993) 2nd Ed., T. E. Creighton, New York; Post-translational Covalent Modifications of Proteins (1983) B. C. Johnson, Ed., Academic Press, New York; Seifter et al. (1990) Analysis for protein modifications and nonprotein cofactors, Meth. Enzymol. 182: 626-646 and Rattan et al., (1992) Protein Synthesis: Post-translational Modifications and Aging, Ann NY Acad Sci, 663: 48-62).

A "hyperglycosylated polypeptide" or "hyperglycosylated analog of a polypeptide" is understood as a polypeptide whose amino acid sequence has been altered in such a way as to possess at least one more additional glycosylation site or a polypeptide with the same amino acid sequence but whose glycosylation level has been increased.

"Isolated polynucleotide" or "isolated polypeptide" is understood as a polynucleotide or a polypeptide such as previously defined which is isolated from the human body or otherwise produced by a technical process.

The term "FVII" or "FVII polypeptide" refers to a Factor VII polypeptide provided in single chain form.

The term "FVIIa" or "FVIIa polypeptide" refers to a Factor VIIa polypeptide provided in its activated two-chain form. When the amino acid sequence SEQ ID NO. 3 is used to describe the amino acid sequence of FVIIa, it is understood that the peptide bond between R152 and I153 of the single chain form has been cleaved, and that one of the chains comprises amino acid residues 1-152, and the other chain comprises amino acid residues 153-406.

"Identity" is understood as the measurement of nucleotide or polypeptide sequences identity. Identity is a term well known to a person skilled in the art and well described in the literature (Lesk, A.M., Computational Molecular Biology (1998) Ed., Oxford University Press, New York; Smith, D.W., Biocomputing Informatics and Genome Project, (1993) Ed., Academic Press, New York; Griffin, A.M. and Griffin H.G., Computer Analysis of Sequence Data, Part I, (1994) Ed, Humana Press, New Jersey; and Von Heinje, G., Sequence Analysis in Molecular Biology (1987) Academic Press).

The methods commonly employed to determine the identity and the similarity between two sequences are equally well described in the literature (Martin J. Bishop, Guide to Huge Computer (1994) Ed, Academic Press, San Diego and Carillo H. and Lipton D., Siam J Applied Math (1988) 48: 1073).

A polynucleotide having, for example, an identity of at least 95 % with the nucleotide sequence SEQ ID NO. 1 is a polynucleotide which contains at most 5 points of mutation over 100 nucleotides, compared to said sequence. These points of mutation can be one (or several) substitution(s), addition(s) and/or deletion(s) of one (or several) nucleotide(s).

In the same way, a polypeptide having, for example, an identity of at least 95 % with the amino acid sequence SEQ ID NO. 2 is a polypeptide that contains at most 5 points of mutation over 100 amino acids, compared to said sequence.

These points of mutation can be one (or several) substitution(s), addition(s) and/or deletion(s) of one (or several) amino acid(s).

The polynucleotides and the polypeptides according to the invention which are not fully identical over the nucleotide sequences SEQ ID NO. 1 or SEQ ID NO.2 or the amino acid sequences SEQ ID NO. 3 and SEQ ID NO.4, it being understood that these sequences contains at least one of the above selected SNPs, are considered as variants according to the invention.

A polypeptide according to the invention generally possesses the same or practically the same biological activity as the protein encoded by the amino acid sequence SEQ ID NO. 3 or SEQ ID NO.4. This biological activity may although be increased or lowered depending on the SNPs present on the polypeptide according to the invention.

A polypeptide according to the invention generally possesses the same or practically the same biological half-life as the protein encoded by the amino acid sequence SEQ ID NO. 3 or SEQ ID NO.4. This biological half-life may although be increased or lowered depending on the SNPs present on the polypeptide according to the invention.

A polypeptide according to the invention, may be obtained by site-directed mutagenesis from FVII wild type sequences or by direct synthesis.

"SNP" is understood as a natural variation of a base in a nucleotide sequence or an amino acid in a peptide sequence. A SNP on a nucleotide sequence can be coding, silent or non-coding depending on the consequences it induces on the protein translation.

A coding SNP is a polymorphism included in the coding sequence of a nucleotide sequence that involves a modification of an amino acid in the sequence of amino acids encoded by this nucleotide sequence. In this case, the term SNP applies equally, by extension, to a mutation in an amino acid sequence.

A silent SNP is a polymorphism included in the coding sequence of a nucleotide sequence that does not involve a modification of an amino acid in the amino acid sequence encoded by this nucleotide sequence.

A non-coding SNP is a polymorphism included in the non-coding sequence of a nucleotide sequence. This polymorphism can notably be found in an intron, a splicing zone, a transcription promoter or an enhancer site sequence.

"Functionality" is understood as the biological activity and/or half-life of a polypeptide or of a polynucleotide.

"Functional SNP" is understood as a SNP, such as previously defined, which does not impair the functionality of a polypeptide or a polynucleotide. The functionality of said polypeptide may consist in the conserved, reduced, suppressed or increased biological activity and/or half-life of the wild-type reference gene or polypeptide.

The functionality of a polypeptide or of a polynucleotide according to the invention may also consist of a change in the profile of said biological activity, i.e. acquisition or loss of biological properties.

The biological activity may, for instance, be linked to the affinity or to the absence of affinity of a polypeptide with other blood coagulation factors.

### Polynucleotides.

The present invention has for its first object an isolated polynucleotide comprising:
a) a nucleotide sequence having at least 80% identity with the nucleotide sequence SEQ ID NO. 2 (Factor VII coding sequence), and comprising at least one SNP selected from c512t, t599g, g823a, g851a, g1060t and c1151t; or
b) a nucleotide sequence complementary to a nucleotide sequence under a).
An isolated polynucleotide according to the invention may also comprise:
a) a nucleotide sequence having at least 80% identity with the nucleotide sequence SEQ ID NO. 1 (Factor Vil whole gene sequence) and comprising at least one SNP selected from c9951t, t11004g, g12640a, g12668a, g12877t and c12968t; or
b) a nucleotide sequence complementary to a nucleotide sequence under a). Preferably, this isolated polynucleotide under a) has at least 85% identity, preferably at least 90%, more preferably 95% and even more preferably 99% identity with the nucleotide sequence SEQ ID NO.1or SEQ ID NO.2.

An isolated polynucleotide according to the invention preferably encodes a polypeptide according to the invention, comprising the amino acid sequence SEQ ID NO. 4 or SEQ ID NO.3 (immature Factor VII amino acid sequence) and having at least one SNP selected from S111F (S171 F), I140S (I200S), E215K (E275K), R224Q (R284K), A294S (A354S) and T324M (T384M).

It is here understood that the positions of S111 F, 1140S, E215K, R224Q, A294S, and T324M SNPs are given relatively to the numbering of the amino acids in SEQ ID NO.4. (and into brackets relative to that in SEQ ID NO.3).

According to a preferred object of the invention, the previously defined polypeptide comprises a single coding SNP according to the invention.

Preferably a polynucleotide according to the invention is composed of a DNA or RNA molecule.

An isolated polynucleotide according to the invention can equally include, for example, nucleotide sequences coding for pre-, pro- or pre-pro-protein amino acid sequences or marker amino acid sequences, such as hexa-histidine peptide.

A polynucleotide of the invention can equally be associated with nucleotide sequences coding for other proteins or protein fragments in order to obtain fusion proteins or other purification products.

A polynucleotide according to the invention can equally include nucleotide sequences such as the 5' and/or 3' non-coding sequences, such as, for example, transcribed or non-transcribed sequences, translated or non-translated sequences, splicing signal sequences, polyadenylated sequences, ribosome binding sequences or even sequences which stabilize mRNA.

A nucleotide sequence is considered as being complementary to a polynucleotide sequence when it can hybridize with a nucleotide sequence under stringent hybridization conditions over its whole length.

"Stringent hybridization conditions" is generally but not necessarily understood as the chemical conditions that permit a hybridization when the nucleotide sequences have an identity of at least 80 %, preferably greater than or equal to 90 %, still more preferably greater than or equal to 95 % and most preferably greater than or equal to 97 %.

These stringent conditions can be obtained according to methods well known to a person skilled in the art and, for example, by an incubation of the polynucleotides, at 42° C, in a solution comprising 50 % formamide, 5xSSC (150 mM of Nacl, 15 mM of trisodium citrate), 50 mM of sodium phosphate (pH = 7.6), 5x Denhardt Solution, 10 % dextran sulfate and 20 µg denatured salmon sperm DNA, followed by washing the filters at 0.1 x SSC, at 65° C.

Within the scope of the invention, when the stringent hybridization conditions permit hybridization of the nucleotide sequences having an identity more than 99%, preferably equal to 100%, the complementary sequence under b) is considered to be strictly complementary to the nucleotide sequence under a) over its whole length.

It is thus understood within the meaning of the present invention that the nucleotide sequence complementary to a nucleotide sequence under a) as referred to above, comprises at least one anti-sense SNP according to the invention.

According to a preferred embodiment the isolated polynucleotide of the invention comprises:
a) a nucleotide sequence that can hybridize with the complementary nucleotide coding sequence of SEQ ID N0:1 (or SEQ ID NO.2) under stringent conditions, said isolated polynucleotide comprising at least one of the SNPs c9951t (c512t), t11004g (t599g), g12640a (g823a), g12668a (g851 a), g12877t (g1060t) and c12968t (c1151t) and encoding factor VII comprising at least one of the amino acid SNPs S111F, I140S, E215K, R224Q, A294S, and T324M wherein said stringent conditions are an incubation of said polynucleotide with said complementary sequence of SEQ ID NO.1 , at 40 °C, in a solution comprising 50% formamide, 5xSSC, 50 mM of sodium phosphate with pH=7.6, 5x Denhardt Solution, 10% dextran sulfate and 20ug denatured salmon sperm DNA, followed by washing the filters at 0.1 xSSC, at 65 °C ; or
b) a nucleotide sequence complementary to a nucleotide sequence under a) over its whole length.

### Identification, hybridization and/or amplification of a polynucleotide comprising a SNP

The present invention also has for its object the use of all or part of a previously defined polynucleotide, in order to identify, hybridize and/or amplify all or part of a polynucleotide consisting of the nucleotide sequence SEQ ID NO. 1 or SEQ ID NO.2, more particularly including the SNPs described in the present application. A fragment of a polynucleotide according to the invention includes one of the SNPs previously defined. It is preferably composed of 5 to 50 nucleotides and more preferably between 10 to 40 nucleotides.
Such fragments are useful to identify, hybridise and/or amplify a polynucleotide comprising a sequence having at least 80 %, preferably 90%, more preferably 95%, identity with nucleotide sequence SEQ ID NO. 1 or SEQ ID NO.2 (including or not said SNPs).

### Genotyping and determination of the frequency of a SNP

The present invention equally has for its object the use of all or part of a polynucleotide according to the invention for the genotyping and more particularly with respect to nucleotide sequences which have between 90 and 100 % identity with the nucleotide sequence of FVII gene and which comprise at least one of the following SNPs disclosed in the present application.

According to the invention, the genotyping may be carried out on an individual or a population of individuals.

Within the meaning of the invention, genotyping is defined as a process for the determination of the genotype of an individual or of a population of individuals. Genotype consists of the alleles present at one or more specific loci.

"Population of individuals" is understood as a group of at least two determined individuals selected in random or non-random fashion. These individuals can be humans, animals, microorganisms or plants.

A functional SNP according to the invention is preferably genotyped in a population of individuals.

Many technologies exist which can be implemented in order to genotype SNPs (Kwok Pharmacogenomics, "High-throughput genotyping assay approaches" (2000), 1:95-100). These technologies are based on one of the four following principles: allele specific oligonucleotide hybridization, oligonucleotide elongation by dideoxynucleotides optionally in the presence of deoxynucleotides, ligation of allele specific oligonucleotides or cleavage of allele specific oligonucleotides. Each of these technologies can be coupled to a detection system such as measurement of direct or polarized fluorescence, or mass spectrometry.

Genotyping can notably be carried out by minisequencing with hot ddNTPs (2 different ddNTPs labeled by different fluorophores) and cold ddNTPs (2 different non labeled ddNTPs), in connection with a polarized fluorescence scanner. The minisequencing protocol with reading of polarized fluorescence (FP-TDI Technology or Fluorescence Polarization Template-direct Dye-Terminator Incorporation) is well known to a person skilled in the art.

It can be carried out on a product obtained after amplification by polymerase chain reaction (PCR) of the genomic DNA of each individual. This PCR product is selected to cover the polynucleotide genic region containing the studied SNP. After the last step in the PCR thermocycler, the plate is placed on a polarized fluorescence scanner for a reading of the labeled bases by using fluorophore specific excitation and emission filters. The intensity values of the labeled bases are reported on a graph.

For the PCR amplification, in the case of a SNP of the invention, the sense and antisense primers, respectively, can easily be selected by a person skilled in the art according to the position of the SNPs of the invention in the FVII gene nucleotide sequence.

The present invention also concerns the use of a polynucleotide according to the invention for the research of variants in the FVII gene nucleotide sequence.

### SNPs of the invention as genetic markers

Whereas SNPs modifying functional sequences of genes (e.g. promoter, splicing sites, coding region) are likely to be directly related to disease susceptibility or resistance, all SNPs (functional or not) may provide valuable markers for the identification of one or several genes involved in these disease states and, consequently, may be indirectly related to these disease states (See Cargill et al. (1999). Nature Genetics 22:231-238; Riley et al. (2000) Pharmacogenomics 1:39-47; Roberts L. (2000) Science 287: 1898-1899).

Thus, the present invention also concerns a databank comprising at least one of the following SNPs: c9951t, t11004g, g12640a, g12668a, g12877t and c12968t in a polynucleotide sequence, being understood that said SNPs are numbered in accordance with the nucleotide sequence SEQ ID NO. 1.

This databank may be analyzed for determining statistically relevant associations between said SNPs and a disease or a resistance to a disease.

The present invention also concerns the use of at least one of said SNPs for developing diagnostic/prognostic kits for a disease or a resistance to a disease.

A SNP of the invention such as defined above may be directly or indirectly associated to a disease or a resistance to a disease.

Preferably, these diseases are those defined below.

### Expression vector and host cell

The present invention also has for its object a recombinant vector comprising at least one polynucleotide according to the invention.

Numerous expression systems can be used like, for example, chromosomes, episomes, derived viruses. More particularly, the recombinant vectors used can be derived from bacterial plasmids, transposons, yeast episome, insertion elements, yeast chromosome elements, viruses such as baculovirus, papilloma viruses such as SV40, vaccinia viruses, adenoviruses, fox pox viruses, pseudorabies viruses, retroviruses.

These recombinant vectors can equally be cosmid or phagemid derivatives. The nucleotide sequence can be inserted in the recombinant expression vector by methods well known to a person skilled in the art such as, for example, those that are described in Sambrook et al., Molecular Cloning : A Laboratory Manual (1989) 2nd Ed., Cold Spring Harbor Laboratory Press, Cold Spring Harbor, N.Y.

The recombinant vector can include nucleotide sequences that control the regulation of the polynucleotide expression as well as nucleotide sequences permitting the expression and the transcription of a polynucleotide of the invention and the translation of a polypeptide of the invention, these sequences being selected according to the host cells that are used.

Thus, for example, an appropriate secretion signal can be integrated in the recombinant vector so that the polypeptide, encoded by the polynucleotide of the invention, will be directed towards the lumen of the endoplasmic reticulum, towards the periplasmic space, on the membrane or towards the extracellular environment.

The present invention also has for its object a host cell comprising a recombinant vector according to the invention.

The introduction of the recombinant vector in a host cell can be carried out according to methods that are well known to a person skilled in the art, such as transfection by calcium phosphate, transfection by DEAE dextran, transfection, microinjection, transfection by cationic lipids, electroporation, transduction or infection.

The host cell can be, for example, bacterial cells such as cells of streptococci, staphylococci, *E*. *coli* or *Bacillus subtilis,* cells of fungi such as yeast cells and cells of *Aspergillus, Streptomyces,* insect cells such as cells of *Drosophilia* S2 and of *Spodoptera* Sf9, animal cells, such as CHO, COS, HeLa, C127, BHK, HEK 293 cells and human cells of the subject to treat or even plant cells.

The host cells can be used, for example, to express a polypeptide of the invention or as active product in pharmaceutical compositions, as will be seen hereinafter.

### Polypeptides.

The present invention has also for object an isolated polypeptide characterized in that it is encoded by an isolated polynucleotide according to the invention as previously defined.

Such an isolated polypeptide preferably comprises an amino acid sequence having at least 80% identity with the amino acid sequence SEQ ID NO. 4 (mature FVII amino acid sequence) or SEQ ID NO.3 (immature FVII amino acid sequence), wherein said polypeptide comprises at least one SNP selected from S111F (S171F), I140S (I200S), E215K (E275K), R224Q (R284K), A294S (A354S) and T324M (T384M).

A preferred isolated polypeptide according the invention has an amino acid sequence at least 85%, preferably at least 90%, more preferably 95% and even more preferably 99% identical with SEQ ID NO.4 or SEQ ID NO.3.

In particular said isolated polypeptide can comprise an amino acid sequence selected from sequences referred to as SEQ ID NO. 5 to 10, or preferably consists in said amino acid sequence.

In a preferred embodiment, the isolated polypeptide according to the invention exhibits the same biological activities as mature FVII protein but to a higher or lower extent, or at least one of said biological activities, in particular that selected among the following:
- FX activation, in particular in a tissue factor independent assay,
- clotting activity, in particular a reduced clotting time when assayed in a whole blood assay;
- Tissue Factor binding affinity,
- proteolytic activity,
- phospholipid membrane binding affinity,

In a preferred embodiment as well, the isolated polypeptide according to the invention exhibits the same half-life profile in vivo as mature FVII protein but to a higher or lower extent, when assayed in animal models (ie., mouse, rat or rabbit) or in humans.

An isolated polypeptide according to the invention is preferably obtained by a method comprising cultivating a host cell as previously defined in a culture medium and separating said polypeptide from the culture medium.

The present invention also concerns a hyperglycosylated analog of a polypeptide according to the invention in order to improve its therapeutic properties.

More preferably, the present invention concerns pegylated analogs of a polypeptide according to the invention.

Indeed, it is known in the art that the oligosaccharide component can significantly affect properties relevant to efficacy of a therapeutic glycoprotein, including physical stability, resistance to protease attack, interactions with the immune system, pharmacokinetics and specific biological activity (Dube et al. (1988) J. Biol. Chem. 263, 17516; Delorme et al. (1992) Biochemistry 31, 9871-9876). Techniques that permit the increase in the number of carbohydrate chains on a protein are well known by the one skilled in the art and can consist for instance in one of the followings:
- introduction of new sites available for glycosylation using site-directed mutagenesis creating amino acid residue substitution or addition (see EP0640619 and U.S. Patent Application No. 09/853731, published as Publication No. 20020037841, for example).
- glycosylation engineering of proteins by using a host cell which harbor the nucleic acid encoding the protein of interest and at least one nucleic acid encoding a glycoprotein-modifying glycosyl transferase as suggested by W09954342 application.

The present invention equally has for its object a process for the preparation of the above-described polypeptide, in which a previously defined host cell is cultivated in a culture medium and said polypeptide is isolated from the culture medium.

The polypeptide can be purified from the host cells' culture medium, according to methods well known to a person skilled in the art such as precipitation with chaotropic agents such as salts, in particular ammonium sulfate, ethanol, acetone or trichloroacetic acid; acid extraction; ion exchange chromatography; phosphocellulose chromatography; hydrophobic interaction chromatography; affinity chromatography; hydroxyapatite chromatography or exclusion chromatographies.

"Culture medium" is understood as the medium in which the polypeptide of the invention is isolated or purified. This medium can be composed of the extracellular medium and/or the cellular lysate. Techniques well known to a person skilled in the art equally permit him or her to give back an active conformation to the polypeptide, if the conformation of said polypeptide was altered during the isolation or the purification.

### Antibodies.

The present invention also has for its object a process for obtaining an immunospecific antibody.

"Antibody" is understood as including monoclonal, polyclonal, chimeric, simple chain, humanized antibodies as well as the Fab fragments, including Fab or immunoglobulin expression library products.

An immunospecific antibody can be obtained by immunization of an animal with a polypeptide according to the invention.

The invention also relates to an immunospecific antibody for a polypeptide according to the invention, such as defined previously.

A polypeptide according to the invention, one of its fragments, an analog, one of its variants or a cell expressing this polypeptide can also be used to produce immunospecific antibodies.

The term "immunospecific" means that the antibody possesses a better affinity for the polypeptide of the invention than for other polypeptides known in the prior art.

The immunospecific antibodies can be obtained by administration of a polypeptide of the invention, of one of its fragments, of an analog or of an epitopic fragment or of a cell expressing this polynucleotide in a mammal, preferably non human, according to methods well known to a person skilled in the art.

For the preparation of monoclonal antibodies, typical methods for antibody production can be used, starting from cell lines, such as the hybridoma technique (Kohler et al. (1975) Nature 256: 495-497), the trioma technique, the human B cell hybridoma technique (Kozbor et al. (1983) Immunology Today 4: 72) and the EBV hybridoma technique (Cole et al. (1985) Monoclonal Antibodies and Cancer Therapy, pp. 77-96, Alan R. Liss).

The techniques of single chain antibody production such as described, for example, in US Patent No. 4,946,778 can equally be used.

Transgenic animals such as mice, for example, can equally be used to produce humanized antibodies.

### Agents interacting with the polypeptide of the invention

The present invention equally has for its object a process for the identification of an agent activating or inhibiting a polypeptide according to the invention, comprising:
a) the preparation of a recombinant vector comprising a polynucleotide according to the invention containing at least one coding SNP of the invention,
b) the preparation of host cells comprising a recombinant vector according to a),
c) the contacting of host cells according to b) with an agent to be tested, and
d) the determination of the activating or inhibiting effect generated by the agent to test.

A polypeptide according to the invention can also be employed for a process for screening compounds that interact with it.

These compounds can be activating (agonists) or inhibiting (antagonists) agents of intrinsic activity of a polypeptide according to the invention. These compounds can equally be ligands or substrates of a polypeptide of the invention (Coligan et al. (1991) Current Protocols in Immunology 1 (2), Chap. 5). They can also be inhibitors of a polypeptide of the invention.

In general, in order to implement such a process, it is first desirable to produce appropriate host cells that express a polypeptide according to the invention. Such cells can be, for example, cells of mammals, yeasts, insects such as *Drosophilia* or bacteria such as *E*. *coli.*

These cells or membrane extracts of these cells are then placed in the presence of compounds to be tested.

The binding capacity of the compounds to be tested with the polypeptide of the invention can then be observed, as well as the inhibition or the activation of the functional response of the polypeptide of the invention.

Step d) of the above process can be implemented by using an agent to be tested that is directly or indirectly labeled. It can also include a competition test, by using a labeled or non-labeled agent and a labeled competitor agent.

It can equally be determined if an agent to be tested generates an activation or inhibition signal on cells expressing the polypeptide of the invention by using detection means appropriately chosen according to the signal to be detected.

Such activating or inhibiting agents can be polynucleotides, and in certain cases oligonucleotides or polypeptides, such as proteins or antibodies, for example.

The present invention also has for its object a process for the identification of an agent activated or inhibited by a polypeptide according to the invention, comprising:
a) the preparation of a recombinant vector comprising a polynucleotide according to the invention containing at least one coding SNP of the invention,
b) the preparation of host cells comprising a recombinant vector according to a),
c) placing the host cells according to b) in the presence of an agent to be tested, and
d) the determination of the activating or inhibiting effect generated by the polypeptide according to the invention on the agent to be tested.

An agent activated or inhibited by the polypeptide of the invention is an agent that responds, respectively, by activation or inhibition in the presence of this polypeptide. The agents, activated or inhibited directly or indirectly by the polypeptide of the invention, can consist of polypeptides such as, for example, membranal or nuclear receptors, kinases and more preferably tyrosine kinases, transcription factor or polynucleotides.

### Detection of diseases

The present invention also has for object a method for diagnosing or determining a prognosis of coagulation related disorders or a resistance to said disorders, comprising detecting *in-vitro* at least one SNP selected from the group consisting of c9951t, t11004g, g12640a, g12668a, g12877t and c12968t, in the gene encoding Factor VII.

This method may permit genetic diagnosis and/or determination of whether a patient is affected or at risk of being affected or, to the contrary, presents a partial resistance to the development of a disease, an indisposition or a disorder linked to the presence of a polynucleotide according to the invention and/or a polypeptide according to the invention.

This process also permits genetic diagnosis of a disease or resistance to a disease linked to the presence, in a subject, of the mutant allele encoded by a SNP according to the invention.

Many methods well known to a person skilled in the art can be used to determine the expression of a polynucleotide of the invention and to identify the genetic variability of this polynucleotide (Chee et al. (1996) Science, 274:610-613).

For instance, the level of expression of the polynucleotide may be measured by quantifying the level of RNA encoded by this polynucleotide (and coding for a polypeptide) according to methods well known to a person skilled in the art as, for example, by PCR, RT-PCR, RNase protection, Northern blot, and other hybridization methods.

Also, the presence or the absence as well as the concentration of a polypeptide according to the invention in a patient or a sample from a patient may be carried out by well known methods such as, for example, by radioimmunoassay, competitive binding tests, Western blot and ELISA tests.

Subsequently, the determined concentration of the polypeptide according to the invention can be compared with the natural wild-type FVII protein concentration usually found in a patient.

A person skilled in the art can identify the threshold above or below which appears the sensitivity or, to the contrary, the resistance to the disease, the indisposition or the disorder evoked above, with the help of prior art publications or by conventional tests or assays, such as those cited in the present specification.

### Therapeutic compounds and treatments of diseases

The present invention also has for its object a therapeutic compound containing, by way of active agent, a polypeptide according to the invention as defined above.

The invention also relates to the use of a polypeptide according to the invention for the manufacture of a therapeutic compound intended for the prevention or the treatment of different human disorders and/or diseases. These diseases can be disorders and/or human diseases, such as cancers and tumors, infectious diseases, venereal diseases, immunologically related diseases and/or autoimmune diseases and disorders, cardiovascular diseases, metabolic diseases, central nervous system diseases, gastrointestinal disorders, and disorders connected with chemotherapy treatments.

Said cancers and tumors include carcinomas comprising metastasizing renal carcinomas, melanomas, lymphomas comprising follicular lymphomas and cutaneous T cell lymphoma, leukemias comprising chronic lymphocytic leukemia and chronic myeloid leukemia, cancers of the liver, neck, head and kidneys, multiple myelomas, carcinoid tumors and tumors that appear following an immune deficiency comprising Kaposi's sarcoma in the case of AIDS.

Said infectious diseases include viral infections comprising chronic hepatitis B and C and HIV/AIDS, infectious pneumonias, and venereal diseases, such as genital warts.

Said immunologically and auto-immunologically related diseases may include the rejection of tissue or organ grafts, allergies, asthma, psoriasis, rheumatoid arthritis, multiple sclerosis, Crohn's disease and ulcerative colitis.

Said cardiovascular diseases may include brain injury and anemias including anemia in patients under dialysis in renal insufficiency, as well as anemia resulting from chronic infections, inflammatory processes, radiotherapies, and chemotherapies.

Said metabolic diseases may include such non-immune associated diseases as obesity.

Said diseases of the central nervous system may include Alzheimer's disease, Parkinson's disease, schizophrenia and depression.

Said diseases and disorders may also include wound healing and osteoporosis.

The polynucleotides and polypeptides according to the invention are preferably intended for preventing or treating coagulation related disorders, more particularly in haemophiliac persons, persons having defective blood coagulation or having defective Factor VII.

By "coagulation related disorders" is meant any situation where blood coagulation is desired. This comprises for instance situation wherein the bleeding is associated with trauma, intracerebral hemorrhage, traumatic brain injury, burns, variceal bleeds, gastrointestinal bleeding, hepatic rejection, thrombocytopenia, cardiac, thoracic aortic, or spinal surgery, organ transplantation, fibrinolytic treatment, anticoagulant treatment, hysterectomy or postpartum bleeding.

Such a treatment could benefit for instance to:
- patients with congenital haemophilia A or B with inhibitors or factor VII deficiency;
- patients suffering from a Glanzmann's thrombasthenia with antibodies anti-GP IIb-IIa and/or anti-HLA ;
- patients with acquired haemophilia in the prevention of bleeding in surgical interventions or invasive procedures in patients with haemophilia A or B.
- patients in situation of emergency medicine when severe bleedings need to be stopped as fast as possible.

When bleeding needs to be treated in patients with defective coagulation, the active agents according to the invention are preferably administered through intravenous bolus injection.

Typically the initial dose varies from 20 to 40 µg/kg of body weight for non emergent anticoagulant reversal and from 40 to 90 µg/kg of body weight in other cases. Injections may be repeated, following the initial dose.

The duration of the treatment and the interval between successive injections vary with the severity of the hemorrhage, the invasive procedure, the surgery being performed or the severity of the trauma. Initially, the dosing interval is 2-3 hours to achieve hemostasis. This interval can be increased, once effective hemostasis is achieved to every four, six, eight or twelve hours for as long as treatment is indicated.

The polypeptide according to the invention can also be administered by contact, for instance under the form of a powder or spray composition, more especially in situation of emergency medicine when severe bleedings need to be stopped as fast as possible.

Certain of the compounds permitting to obtain the polypeptide according to the invention as well as the compounds obtained or identified by or from this polypeptide can likewise be used for the therapeutic treatment of the human body, i.e. as a therapeutic compound.

This is why the present invention also has for an object a therapeutic compound containing, by way of active agent, a polynucleotide according to the invention containing at least one previously defined SNP, a previously defined recombinant vector, a previously defined host cell, and/or a previously defined antibody.

When it is used as active agent, a polypeptide according to the invention is generally administered at doses ranging between 1 and 300 units/kg of the subject.

The invention also has as an object a pharmaceutical composition that contains, as active agent, at least one above-mentioned compound such as a polypeptide according to the invention; a hyperglycosylated analog of a polypeptide according to the invention, a fusion protein, hyperglycosylated or not, containing a polypeptide according to the invention; a polynucleotide according to the invention containing at least one previously defined SNP of the invention, a previously defined recombinant vector, a previously defined host cell, and/or a previously defined antibody, as well as a pharmaceutically acceptable excipient.

In these pharmaceutical compositions, the active agent is advantageously present at physiologically effective doses.

These pharmaceutical compositions can be, for example, solids or liquids and be present in pharmaceutical forms currently used in human medicine such as, for example, simple or coated tablets, gelcaps, granules, caramels, suppositories and preferably injectable preparations and powders for injectables. These pharmaceutical forms can be prepared according to usual methods.

The active agent(s) can be incorporated into excipients usually employed in pharmaceutical compositions such as talc, Arabic gum, lactose, starch, dextrose, glycerol, ethanol, magnesium stearate, cocoa butter, aqueous or nonaqueous vehicles, fatty substances of animal or vegetable origin, paraffinic derivatives, glycols, various wetting agents, dispersants or emulsifiers, preservatives.

The active agent(s) can also be incorporated into nanoparticules for a slow release into the body.

The active agent(s) can be administered systemically in the body or locally at the site(s) of the bleeding.

The active agent(s) according to the invention can be employed alone or in combination with other compounds such as therapeutic compounds such as cytokines or growth factors, for example.

The different formulations of the pharmaceutical compositions are adapted according to the mode of administration.

The pharmaceutical compositions can be administered by different routes of administration known to a person skilled in the art.

### Variants and equivalents

The present invention also concerns variant or equivalent proteins having a biological activity substantially similar or higher than the polypeptides according to the invention.

These variants can be obtained, for instance by post-translational modifications.

Such variants, like the polypeptides of the present invention, may be selected for their different biological activities in relation with blood coagulation.

For assessing the functionality of FVII protein and variants, the following biological activity tests are particularly useful:
- The activity of plasma coagulation, which can be determined with commercial kits including sera lacking FVII, like the coagulation kit Staclot VIIa-rTF designed by Diagnostica Stago.
- The FVIIa protease activity, which may be monitored according to standard Bradford protocols using for instance the S-2288 (Chromogenix) as substrate oligopeptides.
- Measurement of the half-life of the compound upon injection of the molecule in rats and monitoring of the protein clearance using Elisa techniques.

Additionally, the results of these activity tests may be compared with those obtained with wild-type FVIIa product, which is available on the market under the trademark Novoseven® (NovoNordisk).

## Claims

**1.** An isolated polynucleotide comprising:
a) a nucleotide sequence:
- having at least 80% identity with the nucleotide sequence SEQ ID NO. 2, and
- comprising at least one SNP selected from c512t, t599g, g823a, g851a, g1060t and c1151t; or
b) a nucleotide sequence complementary to a nucleotide sequence under a).

**2.** An isolated polynucleotide according to claim 1, **characterized in that** the nucleotide sequence under a) has at least 90% identity, preferably at least 95%, more preferably 99% identity with the nucleotide sequence SEQ ID NO.2.

**3.** An isolated polynucleotide comprising:
a) a nucleotide sequence:
- having at least 80% identity with the nucleotide sequence SEQ ID NO. 1 encoding FVII protein, and
- comprising at least one SNP selected from c9951t, t11004g, g12640a, g12668a, g12877t and c12968t; or
b) a nucleotide sequence complementary to a nucleotide sequence under a).

**4.** An isolated polynucleotide according to claim 1, **characterized in that** the nucleotide sequence under a) has at least 90% identity, preferably at least 95%, more preferably 99% identity with the nucleotide sequence SEQ ID N0.1.

**5.** An isolated polynucleotide according to any one of claims 1 to 4, **characterized in that** it encodes a polypeptide comprising the amino acid sequence SEQ ID NO. 4 and having at least one SNP selected from S111 F, I140S, E215K, R224Q, A294S, and T324M.

**6.** An isolated polynucleotide according to any one of claims 1 to 5, **characterized in that** it encodes a polypeptide comprising amino acids of sequence SEQ ID NO. 3 and having at least one SNP selected from S111F (S171F), 1140S (1200S), E215K (E275K), R224Q (R284K), A294S (A354S) and T324M (T384M)

**7.** Use of an isolated polynucleotide according to any one of claims 1 to 6, or a sequence of said isolated polynucleotide composed of 10 to 40 nucleotides, in order to identify, hybridise and/or amplify a polynucleotide comprising a sequence having at least 80 % identity with nucleotide sequence SEQ ID NO. 1.

**8.** A recombinant vector, **characterized in that** it comprises a polynucleotide according to any one of claims 1 to 6.

**9.** A host cell, **characterized in that** it comprises a recombinant vector according to claim 8.

**10.** An isolated polypeptide **characterized in that** it is encoded by the isolated polynucleotide of any one of claims 1 to 6.

**11.** An isolated polypeptide comprising an amino acid sequence having at least 80% identity with the amino acid sequence SEQ ID NO. 4, wherein said polypeptide comprises at least one SNP selected from S111F, I140S, E215K, R224Q, A294S, and T324M.

**13.** An isolated polypeptide according to claim 11, **characterized in that** its amino acid sequence has at least 90%, preferably at least 95% and more preferably 99% identity with SEQ ID NO.4.

**14.** An isolated polypeptide comprising an amino acid sequence having at least 80% identity with the amino acid sequence SEQ ID NO. 3, wherein said polypeptide comprises at least one SNP selected from S111 F (S171 F), 1140S (1200S), E215K (E275K), R224Q (R284K), A294S (A354S) and T324M (T384M).

**15.** An isolated polypeptide according to claim 14, **characterized in that** its amino acid sequence has at least 90%, preferably at least 95% and more preferably 99% identity with SEQ ID NO.3.

**16.** A method for preparing a polypeptide according to any one of claims 10 to 15, comprising cultivating a host cell according to claim 9 in a culture medium and separating said polypeptide from the culture medium.

**17.** A therapeutic agent comprising one or more compounds selected from the group consisting of: an isolated polynucleotide according to any one of claims 1 to 6; a recombinant vector according to claim 8; a host cell according to claim 9; an isolated polypeptide according to any one of claims 10 to 15.

**18.** Use of one or more compounds selected from the group consisting of: an isolated polynucleotide according to any one of claims 1 to 6; a recombinant vector according to claim 8; a host cell according to claim 9; an isolated polypeptide according to any one of claims 10 to 15; for preparing a medicament for the prevention or the treatment of bleedings or hemorrhages.

**19.** Use of one or more compounds selected from the group consisting of: an isolated polynucleotide according to any one of claims 1 to 6; a recombinant vector according to claim 8; a host cell according to claim 9; an isolated polypeptide according to any one of claims 10 to 15 for preparing a medicament for treating coagulation related disorders.

**20.** Use according to claims 17 or 18, wherein said medicament is useful for treating hemophiliac persons, persons having defective blood coagulation or having defective Factor VII.

**21.** A method for diagnosing or determining a prognosis of coagulation related disorders or a resistance to said disorders, comprising detecting in-vitro at least one SNP selected from the group consisting of c9951t, t11004g, g12640a, g12668a, g12877t and c12968t, in the gene encoding Factor VII.
